# EUROPÄISCHE PATENTSCHRIFT

(11) **EP 3 890 949 B1**
(45) Veröffentlichungstag und Bekanntmachung des Hinweises auf die Patenterteilung: **19.04.2023**
(21) Anmeldenummer: 19816689.4
(22) Anmeldetag: 06.12.2019
(51) Int. Cl.: B29C 64/343, B29C 64/112

(54) **VERFAHREN UND VORRICHTUNG ZUR HERSTELLUNG EINES DREIDIMENSIONALEN OBJEKTS**
METHOD AND DEVICE FOR PRODUCING A THREE-DIMENSIONAL OBJECT
PROCÉDÉ ET DISPOSITIF DE FABRICATION D'UN OBJET TRIDIMENSIONNEL

(30) Priorität: 07.12.2018 CH 15162018
(43) Veröffentlichungstag der Anmeldung: 13.10.2021
(73) Patentinhaber: Chemspeed Research AG, 4410 Liestal (CH)
(72) Erfinder: GUELLER, Rolf, 5027 Herznach (CH)
(74) Vertreter: Bohest AG
(86) Internationale Anmeldenummer: PCT/EP2019/084052
(87) Internationale Veröffentlichungsnummer: WO 2020/115308

(56) Entgegenhaltungen:
- KR-A- 20110 060 276
- NL-A- 2 017 088
- US-A1- 2004 231 593
- US-A1- 2006 211 080
- Andrew Alliance: "Andrew, the Pipetting Robot Inside Out", YouTube , 29. Januar 2015 (2015-01-29), XP054979433, Gefunden im Internet: URL:https://www.youtube.com/watch?v=952yeU 13NQA [gefunden am 2019-05-15]

## Beschreibung

Die vorliegende Erfindung betrifft ein Verfahren und eine Vorrichtung zur Herstellung und/oder Bearbeitung eines dreidimensionalen Objekts mit einem Druckmaterial, das an einer Zielposition in Form von diskreten dreidimensionalen Druckmaterialelementen abgegeben wird.

Die gängigerweise als dreidimensionales Drucken bzw. 3D-Drucken bezeichnete additive Fertigung dreidimensionaler Objekte ist eine sich schnell weiterentwickelnde Fertigungstechnologie mit einer wachsenden Vielzahl von Anwendungsmöglichkeiten, die sowohl private Anwendungen (z.B. in den Bereichen Kunst, Modellbau, Schmuck, Mode, ...) als auch industrielle Anwendungen (z.B. Fast-Prototyping, aber auch Herstellung von Serieteilen bis hin zur Herstellung von Nahrungsmitteln) in den verschiedensten Industriesparten, Wissenschaft und Forschung bis hin zu Medizin und Biotechnologie (z.B. Fertigung von auf einzelne Patienten abgestimmte Prothesen oder gar Ersatzorganen aus Zellkulturen) umfasst. Dreidimensionale Druckvorrichtungen werden gemeinhin als 3D-Drucker bezeichnet.

Ein typischer 3D-Drucker ist beispielsweise im Dokument NL 2 017 088 A beschrieben. Dieser 3D-Drucker umfasst einen Druckkopf, der mittels eines Roboterarms an beliebige Zielpositionen innerhalb eines Arbeitsraums bewegt werden kann, um dort Druckmaterial zu deponieren. Das Druckmaterial wird dem Druckkopf aus einer auf dem Roboterarm angeordneten (und entsprechend mitbewegten) Speiseeinheit zugeführt. Alternativ kann die Speiseeinheit auch an oder nahe der Grundplatte der Vorrichtung ruhend angeordnet sein, wobei sie dann über entsprechende Leitungen mit dem Druckkopf verbunden wäre.

Eine Vorrichtung zur Herstellung von primär zweidimensionalen Mikro-Strukturen auf einem Substrat ist im Dokument US 2004/0231593 A1 offenbart. Die Vorrichtung enthält als wesentlichsten Bestandteil einen Applikationskopf, der zur Abgabe von dosierten Tröpfchen ausgebildet ist und piezo-elektrisch nach dem Ink-Jet-Prinzip arbeitet. Der Applikationskopf kann computergesteuert in zwei Dimensionen an jeden beliebigen Zielort der Unterlage bewegt werden, um dort Druckmaterial tröpfchenweise zu deponieren. Die Zufuhr von Druckmaterial in den Applikationskopf erfolgt entweder aus einem am Applikationskopf angebauten Vorratsbehälter und/oder über eine in den Applikationskopf führende Zufuhrleitung. Die Vorrichtung kann auch mehrere oder wechselbare Applikationsköpfe aufweisen, die auch mit verschiedenen Druckmaterialien gespeist sein können. Ferner ist in diesem Dokument, allerdings nur ganz allgemein, erwähnt, dass Druckmaterialtröpfchen auch aufeinander deponiert werden können, wodurch dreidimensionale (Mikro-)Strukturen erzeugt würden. Ein 3D-Druck im engeren Sinn, also die Erzeugung von makroskopischen dreidimensionalen Objekten, ist in diesem Dokument nicht erwähnt. KR20110060276A offenbart einen Zellkulturapparat mit einer Dosiervorrichtung.

Heutige 3D-Drucker arbeiten meist mit einem Material, aus dem ein dreidimensionales Objekt mittels einem von verschiedenen etablierten Verfahren (z.B. Schmelzschichtverfahren (FDM/FFF), Stereolithografie (SLA), Digital Light Processing (DLP), Selektives Lasersintern (SLS), Selektives (Metall-) Laserschmelzen (SLM) oder Jetting-Verfahren (Multi Jet Fusion, HSS)) aufgebaut wird.

Vermehrt kommen auch 3D-Drucker zum Einsatz, welche mehrere Materialien verwenden können (Multipler 3D-Druck), die auf dem entsprechenden Drucker etwa in Form von mehreren bereits mit den unterschiedlichen Materialien vorgefüllten Kartuschen oder in Form von unterschiedlichen auswählbaren Spulen mit drahtförmigen Druckmaterialien (meist Kunststoffe, aber auch Metalldrähte) vorrätig gehalten und bei Bedarf eingesetzt werden. Diese 3D-Drucker erlauben bereits eine grössere Flexibilität in der Materialzusammensetzung des Endprodukts bzw. des zu druckenden Objekts, doch auch hier ist die Auswahl der verwendbaren Druckmaterialien meist durch die verfügbare Druckmethode eingeschränkt und beschränkt sich oftmals beispielsweise bloss auf die farbliche Unterscheidung der verwendeten Materialien eines gleichen Druckmaterial-Typs oder mehrere sich leicht unterscheidende Materialien (z.B. mit unterschiedlichen Härtegraden nach Aushärtung der Druckmaterialien).

So oder so können aber nicht ad hoc z.B. neuartige oder neu entwickelte Materialien eingesetzt werden, weil in der Regel daraus zuerst eine druckbare Formulierung (falls flüssiger Auftrag) oder ein drahtförmiges Material hergestellt werden müsste. Das ist aber für einen ad hoc-Entscheid untauglich und damit z.B. auch in der Forschung und Entwicklung mindestens mit einem enormen Zusatz- bzw. Vorbereitungsaufwand verbunden und nur aufwändig automatisierbar.

Im Bereich Forschung und Entwicklung aber, zum Beispiel bei der Entwicklung neuer geeigneter Materialien für den 3D-Druck, ist es auch deshalb ein Nachteil, dass die Druckmaterialien in vorgefüllten Behältern und Dosier-Kartuschen oder in einer anderen vorbereiteten Form vorrätig gehalten werden müssen, weil neu entwickelte Materialien oft nur in geringen Mengen synthetisiert und/oder formuliert werden können, das Material gar nicht in der gewünschten Form formuliert werden kann (z.B. weil es als schlecht lösliches Pulver vorliegt), weil aus einer neuartigen Legierung zuerst ein als Druckmaterial dienender Draht hergestellt werden müsste, oder einfach, weil das (manuelle) Befüllen der geeigneten Dosierbehälter sehr umständlich und ineffizient ist, wenn man bedenkt, dass so eine Materialprobe nur einmal oder wenige Male für Versuche verwendet wird.

Eine ähnliche Problematik zeigt sich in der Biotechnologie, z.B. bei der Herstellung von künstlichen Organen aus Biomaterialien oder aus gezüchteten Zellen. Auch hier muss das Druckmaterial erst in ausreichender Menge hergestellt und in eine geeignete Dosiervorrichtung (z.B. eine spritzenartige Kartusche) abgefüllt werden, bevor sie im 3D-Drucker zum Einsatz kommen kann. Zudem können lebende Zellen nicht im gefrorenen Zustand aufgetragen werden, was eine Verwendung einer Zellsuspension notwendig macht.

Weiter will ein Wissenschaftler in der Forschung und Entwicklung oft bei jedem aufgetragenen Druckmaterialelement wissen, wieviel aufgetragen wird, und gerade bei neuartigen Materialien, welche sich beim Dosieren anders verhalten, kurz vor dem Auftragen des Druckmaterialelements auf das Druckobjekt entscheiden, ob er dieses Druckmaterialelement aufträgt oder nicht. Hierbei ist eine Entscheidungsmöglichkeit, ob eine genügende Materialmenge abgegeben wird, von Vorteil, und es soll entschieden werden können, ob eine zum Beispiel zu kleine oder zu grosse Materialprobe tatsächlich aufgetragen werden soll oder nicht.

Durch die vorliegende Erfindung sollen nun ein Verfahren und eine entsprechende Vorrichtung für die Herstellung eines dreidimensionalen Objekts zur Verfügung gestellt werden, die die beschriebenen Nachteile der bekannten 3D-Drucker vermeiden und zusätzliche Möglichkeiten speziell in den Bereichen Materialauswahl, Materialvielfalt und der Kombination zwischen verschiedenen Materialien und Materialtypen erlauben.

Vorzugsweise soll die Erfindung einem Forscher ermöglichen, dass er z.B. in einfachen Gläsern vorliegende Substanzen einfach testen kann. Weiter soll er bei bevorzugten Ausführungsvarianten bei jedem aufzutragenden Druckmaterialelement mit einer z.B. vorprogrammierten Entscheidungsfunktion (z.B. "dosieren/auftragen falls Fehler <0.1 mg, sonst verwerfen und neu versuchen") kurz vor dem Auftrag entscheiden können, ob das Druckmaterialelement aufgetragen wird oder nicht. Ausserdem soll es vorzugsweise möglich sein, auch zwei oder mehr Materialien in kleinsten Mengen zu mischen und dann aufzutragen (zum Beispiel ein schlecht lösliches Pulver in einer Flüssigkeit, etc.).

Die der Erfindung zugrunde liegende Aufgabe wird durch das erfindungsgemässe, im unabhängigen Patentanspruch 1 definierte Verfahren und durch die im unabhängigen Patentanspruch 19 definierte Vorrichtung gelöst. Besonders vorteilhafte Weiterbildungen und Ausgestaltungen des erfindungsgemässen Verfahrens und der erfindungsgemässen Vorrichtung sind Gegenstand der jeweils abhängigen Patentansprüche.

Hinsichtlich des Verfahrens besteht das Wesen der Erfindung in Folgendem: Zur Herstellung und/oder Bearbeitung eines dreidimensionalen Objekts mit einem Druckmaterial, das an einer Zielposition in Form von diskreten dreidimensionalen Druckmaterialelementen abgegeben wird, wird eine Dosiervorrichtung zu mindestens einem Vorratsbehälter, in welchem ein Druckmaterial vorrätig gehalten ist, bewegt. Mittels der Dosiervorrichtung wird aus diesem mindestens einen Vorratsbehälter Druckmaterial aufgenommen. Die Dosiervorrichtung wird zu einer in allen drei Raumdimensionen definierten Zielposition bewegt und an dieser Zielposition wird mittels der Dosiervorrichtung eine dosierte Menge Druckmaterial auf ein Substrat bzw. ein darauf angeordnetes oder darauf im Aufbau befindliches dreidimensionales Objekt aufgebracht, um ein Druckmaterialelement zu bilden. Das Bilden eines Druckmaterialelements wird solange wiederholt, bis das dreidimensionale Objekt vollständig aufgebaut und/oder bearbeitet ist.

Durch die Verwendung einer Dosiervorrichtung zur Aufnahme, zum Transport und zum Aufbringen von Druckmaterial können praktisch beliebige Druckmaterialien verarbeitet und dementsprechend Objekte mit praktisch beliebigen Druckmaterialien hergestellt und/oder bearbeitet werden.

Vorteilhafterweise werden zwei oder mehrere unterschiedliche Druckmaterialien vorrätig gehalten und unterschiedliche Druckmaterialien ausgewählt und aufgenommen, um das dreidimensionale Objekt mit unterschiedlichen Druckmaterialien aufzubauen und/oder zu bearbeiten.

Bei einer vorteilhaften Ausführungsvariante werden als Druckmaterial bzw. Druckmaterialien Flüssigkeiten, in Flüssigkeiten gelöste oder suspendierte Feststoffe, Zellsuspensionen oder Biomaterialien eingesetzt.

Bei einer weiteren vorteilhaften Ausführungsvariante werden als Druckmaterial bzw. Druckmaterialien einstechbare oder amorphe Feststoffe oder gefrorene Substanzen eingesetzt. Unter einstechbaren bzw. amorphen Feststoffen werden beispielsweise wachsartige Substanzen oder Substanzen wie z.B. Schokolade verstanden.

Bei einer weiteren vorteilhaften Ausführungsvariante werden als Druckmaterial bzw. Druckmaterialien pulverförmige oder granuläre Feststoffe eingesetzt.

Bei einer vorteilhaften Ausführungsvariante wird mittels der Dosiervorrichtung jeweils eine quantitativ einem Druckmaterialelement entsprechende Menge an Druckmaterial aufgenommen. Die von der Dosiervorrichtung abzugebende bzw. aufzubringende Druckmaterialmenge entspricht so gerade der aufgenommenen Druckmaterialmenge.

Bei einer vorteilhaften Ausführungsvariante wird die jeweils aufgenommene Menge an Druckmaterial vor dem Aufbringen auf das Substrat bzw. das darauf im Aufbau befindliche dreidimensionale Objekt gravimetrisch gewogen und anhand des Wägeergebnisses und vorgegebener Kriterien entschieden, ob die aufgenommene Menge ergänzt oder verworfen und eine neue Menge aufgenommen wird. Dies erlaubt, die Qualität des aufzubauenden bzw. zu bearbeitenden dreidimensionalen Objekts zu verbessern.

Bei einer zweckmässigen Ausführungsvariante wird die durch die Dosiervorrichtung in der Zielposition auf das Substrat bzw. das darauf angeordnete oder darauf im Aufbau befindliche dreidimensionale Objekt in Form eines Druckmaterialelements aufgebrachte Druckmaterialmenge durch Applizieren gerichteter Strahlung oder Hitze oder durch ein anderes härtendes oder polymerisierendes Verfahren mit dem Substrat bzw. dem darauf angeordneten oder bereits zum Teil aufgebauten dreidimensionalen Objekt verschmolzen und/oder gehärtet. Dadurch werden die einzelnen Druckmaterialelemente örtlich fixiert.

Mit Vorteil umfasst die Dosiervorrichtung ein auswechselbares Dosierwerkzeug, wobei das Dosierwerkzeug vor einem Wechsel des aufzunehmenden Druckmaterials verworfen und durch ein neues Dosierwerkzeug ersetzt wird. Dadurch werden Kontaminationsprobleme vermieden.

Alternativ wird das Dosierwerkzeug vor einem Wechsel des aufzunehmenden Druckmaterials gereinigt.

Bei einer vorteilhaften Ausführungsvariante erfolgt der Aufbau und/oder die Bearbeitung des dreidimensionalen Objekts teilweise mittels eines zusätzlichen, für sich ebenfalls zur Herstellung und/oder die Bearbeitung eines dreidimensionalen Objekts ausgebildeten Drucksystems, wobei Druckmaterialelemente sowohl mittels der Dosiervorrichtung als auch mittels des zusätzlichen Drucksystems auf das Substrat bzw. das darauf angeordnete oder das darauf im Aufbau befindliche dreidimensionale Objekt aufgebracht werden. Dadurch können z.B. Grundstrukturen des dreidimensionalen Objekts mittels des zusätzlichen Drucksystems aufgebaut werden und zu diesen Grundstrukturen können weitere Strukturen mittels der Dosiervorrichtung hinzugefügt werden, wobei diese weiteren Strukturen z.B. aus Druckmaterialien bestehen können, die mittels des zusätzlichen Drucksystems nicht verarbeitet werden können.

Bei einer vorteilhaften Ausführungsvariante ist mindestens ein Druckmaterial so ausgebildet, dass das auf das Substrat bzw. das darauf angeordnete oder darauf im Aufbau befindliche dreidimensionale Objekt aufgebrachte Druckmaterialelement eine materialabbauende Wirkung aufweist, so dass Material aus dem dreidimensionalen Objekt abgebaut wird. Ein dreidimensionales Objekt kann so auch punktuell abgebaut, d.h. subtraktiv bearbeitet werden. Das erfindungsgemässe Verfahrens kann also nicht nur zur additiven Fertigung verwendet werden, sondern kann bei Bedarf auch subtraktiv eingesetzt werden.

Bei einer zweckmässigen Ausführungsvariante tritt die materialabbauende Wirkung erst nach Aktivierung des Druckmaterialelements auf, insbesondere nach Aktivierung mittels Hitze oder Strahlung. Auf diese Weise können beispielsweise temporäre Stützstrukturen abgebaut werden, nachdem sie nicht mehr gebraucht werden.

Damit das aufgebrachte Druckmaterialelement eine materialabbauende Wirkung aufweist, kann das mindestens eine Druckmaterial beispielsweise als Säure oder Lösungsmittel ausgebildet sein.

Bei einer weiteren vorteilhaften Ausführungsvariante ist mindestens ein Druckmaterial so ausgebildet, dass das auf das Substrat bzw. das darauf angeordnete oder darauf im Aufbau befindliche dreidimensionale Objekt aufgebrachte Druckmaterialelement die physikalischen oder chemischen Eigenschaften des dreidimensionalen Objekts punktuell verändert. Dies kann z.B. genutzt werden, um die Leitfähigkeit von Teilen des dreidimensionalen Objekts, die beispielsweise aus Silizium bestehen, punktuell zu verändern, insbesondere das Silizium zu dotieren.

Bei einer weiteren vorteilhaften Ausführungsvariante werden mittels der Dosiervorrichtung nacheinander unterschiedliche Druckmaterialien aus mindestens zwei Vorratsbehältern aufgenommen, zu einer in allen drei Raumdimensionen definierten Zielposition transportiert und die unterschiedlichen Druckmaterialien dort nacheinander auf das Substrat bzw. das darauf angeordnete oder darauf im Aufbau befindliche dreidimensionale Objekt aufgebracht. Der Aufbau bzw. die Bearbeitung des dreidimensionalen Objekts kann so beschleunigt werden.

Alternativ werden mittels der Dosiervorrichtung nacheinander unterschiedliche Druckmaterialien aus mindestens zwei Vorratsbehältern aufgenommen, zu einer in allen drei Raumdimensionen definierten Zielposition transportiert und dort auf das Substrat bzw. das darauf angeordnete oder darauf im Aufbau befindliche dreidimensionale Objekt aufgebracht, wobei die unterschiedlichen Druckmaterialien vor dem Aufbringen in der Dosiervorrichtung miteinander vermischt werden. Dies kann insbesondere bei Zweikomponentensystemen zu einem beschleunigten Objektaufbau führen.

Bei einer weiteren vorteilhaften Ausführungsvariante weist die Dosiervorrichtung mindestens zwei Dosierkanäle auf, mittels welcher nacheinander oder gleichzeitig Druckmaterial oder Druckmaterialien aus einem oder mehreren Vorratsbehältern aufgenommen und danach zu mindestens einer in allen drei Raumdimensionen definierten Zielposition transportiert und dort auf das Substrat bzw. das darauf angeordnete oder darauf im Aufbau befindliche dreidimensionale Objekt aufgebracht wird bzw. werden. Der Aufbau bzw. die Bearbeitung des dreidimensionalen Objekts kann so beschleunigt werden.

Hinsichtlich der Vorrichtung besteht das Wesen der Erfindung in Folgendem: Eine Vorrichtung zur Herstellung und/oder Bearbeitung eines dreidimensionalen Objekts mit einem Druckmaterial umfasst eine Einrichtung zur Abgabe von Druckmaterial in Form von diskreten dreidimensionalen Druckmaterialelementen an einer Zielposition.

Sie umfasst eine Dosiervorrichtung, welche dazu ausgebildet ist, aus mindestens einem Vorratsbehälter, in welchem ein Druckmaterial vorrätig gehalten ist, Druckmaterial aufzunehmen. Sie umfasst weiter eine Transporteinrichtung, die dazu ausgebildet ist, die Dosiervorrichtung zu dem mindestens einen Vorratsbehälter zu bewegen und zu einer in allen drei Raumdimensionen definierten Zielposition zu transportieren . Die Dosiervorrichtung ist weiters dazu ausgebildet, das von ihr aufgenommene Druckmaterial in einer dosierten Menge auf ein Substrat bzw. ein darauf angeordnetes oder ein darauf im Aufbau befindliches dreidimensionales Objekt aufzubringen.

Die Dosiervorrichtung zur Aufnahme, zum Transport und zum Aufbringen von Druckmaterial erlaubt die Verarbeitung praktisch beliebiger Druckmaterialien und dementsprechend die Herstellung und/oder Bearbeitung von Objekten aus praktisch beliebigen Druckmaterialien.

Vorteilhafterweise weist die Dosiervorrichtung mindestens ein in einem Halter bereitgestelltes Dosierwerkzeug auf, welches dazu ausgebildet ist, in einen Dosierkopf der Dosiervorrichtung eingesetzt und wieder aus diesem entfernt zu werden. Die Transporteinrichtung ist dabei dazu ausgebildet, den Dosierkopf zu dem im Halter bereitgestellten Dosierwerkzeug zu bewegen und dieses in den Dosierkopf einzusetzen. Ferner ist die Transporteinrichtung dazu ausgebildet, die Dosiervorrichtung zusammen mit dem eingesetzten Dosierwerkzeug über den Vorratsbehälter zu bewegen und das Dosierwerkzeug in das darin vorrätig gehaltene Druckmaterial einzutauchen, wobei eine definierte Menge des Druckmaterials vom Dosierwerkzeug aufnehmbar ist. Durch die Verwendung von in den Dosierkopf einsetzbaren Dosierwerkzeugen kann für jedes Druckmaterial und je nach Grösse des abzugebenden Druckmaterialelements immer ein optimales Dosierwerkzeug benutzt werden.

Vorzugsweise umfasst die Transporteinrichtung einen von einem Steuerungsrechner gesteuerten Handhabungsroboter. Der Handhabungsroboter ist dabei vorteilhafterweise so ausgebildet, dass er eine räumliche Positioniergenauigkeit von mindestens 100-200 µm, vorzugsweise bis hinunter auf 1-2 µm, erreicht.

Bei vorteilhaften Ausführungsvarianten umfasst die Vorrichtung mehrere Vorratsbehälter für gleiche oder unterschiedliche Druckmaterialien, wobei die Vorratsbehälter durch Einzelbehälter oder eine Platte, die vorzugsweise mehrere Vertiefungen aufweist, gebildet sind, insbesondere aus Glas oder Kunststoff. Die Platte kann auch ohne Vertiefungen als ein oder mehrere Behälter für auf ihr angehäufte Druckmaterialien wirken.

Bei einer vorteilhaften Ausführungsvariante umfasst die Vorrichtung mehrere im Halter bereitgestellte Dosierwerkzeuge und sind die Dosierwerkzeuge als Röhrchen bzw. Kapillaren unterschiedlicher Grösse ausgebildet, in welche Druckmaterial eingebracht und wieder aus ihnen abgegeben werden kann. Durch die Bereitstellung mehrerer unterschiedlicher Dosierwerkzeuge kann jeweils ein optimal geeignetes ausgewählt werden.

Vorteilhafterweise weisen die Röhrchen einen in ihnen beweglichen Kolben auf, wobei durch Einsaugen von flüssigem Druckmaterial durch Zurückziehen des Kolbens eine definierte Menge an Druckmaterial aufnehmbar und durch Vorwärtsbewegen des Kolbens aus dem Röhrchen wieder abgebbar ist. Vorzugsweise ist der Kolben länger als das Röhrchen und ragt am oberen Ende des Röhrchens aus diesem heraus.

Vorteilhafterweise weisen die Röhrchen einen in ihnen beweglichen Kolben auf, wobei durch Einstechen eines Röhrchens in festes oder amorphes Druckmaterial oder Pulver oder Granulat eine definierte Menge an Druckmaterial aufnehmbar und durch Vorwärtsbewegen des Kolbens aus dem Röhrchen wieder abgebbar ist. Vorzugsweise ist der Kolben länger als das Röhrchen und ragt am oberen Ende des Röhrchens aus diesem heraus, wobei er vor dem Einstechen im Röhrchen zurückgezogen ist und so beim Einstechen des Röhrchens in festes oder amorphes Druckmaterial oder Pulver oder Granulat die Aufnahme einer definierten Menge an Druckmaterial erlaubt, welche dann durch Vorwärtsbewegen des Kolbens wieder aus dem Röhrchen ausstossbar ist. Der Kolben kann dabei lose im Röhrchen beweglich sein und muss in diesem nicht dicht gleiten bzw. dieses nicht abdichten.

Röhrchen als Dosierwerkzeuge haben sich in anderen Anwendungen bewährt und sind für die erfindungsgemässe Vorrichtung optimal geeignet. Alternativ können die Dosierwerkzeuge auch als Wegwerf-Spritzen ausgebildet sein. Auch diese sind für die erfindungsgemässe Vorrichtung gut geeignet.

Bei anderen vorteilhaften Ausführungsvarianten können anstelle von Röhrchen auch zylindrische, stabförmige, kugelförmige oder anders geformte Festkörper verwendet werden, die speziell in flüssiges, aber auch in pulverförmiges Druckmaterial eingetaucht werden, so dass ein kleiner Tropfen oder eine kleine Pulvermenge am Dosierwerkzeug anhaften bleibt, welches dann plaziert werden kann. Bevorzugt sind dabei die Dosierwerkzeuge als Stäbchen unterschiedlicher Grösse ausgebildet, an deren einem Ende beim Eintauchen in Druckmaterial Druckmaterial haften bleibt. Dies ist speziell bei besonders kleinen Dosiermengen nützlich und erlaubt eine sehr feine Auflösung und sehr kleine Strukturen.

Bei einer vorteilhaften Ausführungsvariante kann die Vorrichtung auch mit einer Heizung oder Strahlungsquelle zum Beheizen des Dosierwerkzeugs im in den Druckkopf eingesetzten Zustand ausgestattet sein. Dadurch kann die im Dosierwerkzeug enthaltene Menge des Druckmaterials z.B. in geschmolzenem Zustand gehalten werden, während sie zur Zielposition bzw. dem Auftragungspunkt transportiert wird.

Zweckmässigerweise weist die Vorrichtung mindestens eine Strahlungsquelle zur punktgenauen Beaufschlagung der auf das Substrat bzw. das darauf angeordnete oder darauf im Aufbau befindliche dreidimensionale Objekt aufgebrachten Druckmaterialmenge auf. Damit können die aufgetragenen Druckmaterialelemente geschmolzen, ausgehärtet oder vernetzt und örtlich fixiert werden.

Vorteilhafterweise ist die mindestens eine Strahlungsquelle am Dosierkopf angeordnet. Dadurch fällt die aufwändige Ausrichtung der Strahlungsquelle auf die Zielposition weg.

Zweckmässigerweise ist das Dosierwerkzeug so ausgebildet, dass die von der mindestens einen Strahlungsquelle ausgehende Strahlung durch das Dosierwerkzeug hindurch zu der aufgebrachten Druckmaterialmenge leitbar ist.

Mit Vorteil ist die mindestens eine Strahlungsquelle für das Schmelzen oder Härten von Druckmaterial ausgebildet.

Vorteilhafterweise umfasst die Vorrichtung zwei oder mehrere Vorratsbehälter für unterschiedliche Druckmaterialien und ist dazu ausgebildet, Druckmaterial aus unterschiedlichen Vorratsbehältern auszuwählen und in das Dosierwerkzeug aufzunehmen, wobei das dreidimensionale Objekt aus zwei oder mehreren unterschiedlichen Druckmaterialien aufbaubar ist.

Bei einer vorteilhaften Ausführungsvariante umfasst die Vorrichtung mindestens ein zusätzliches eigenständiges Drucksystem, welches für sich ebenfalls zur Herstellung und/oder Bearbeitung eines dreidimensionalen Objekts ausgebildet ist. Dabei ist die Vorrichtung vorzugsweise dazu ausgebildet, das dreidimensionale Objekt teilweise mittels der Dosiervorrichtung und teilweise mittels des zusätzlichen Drucksystems aufzubauen und/oder zu bearbeiten. Dadurch können z.B. Teilstrukturen des Objekts mittels des zusätzlichen Drucksystems aufgebaut und/oder bearbeitet werden und zu diesen Teilstrukturen können weitere Strukturen mittels der Dosiervorrichtung hinzugefügt werden, wobei diese weiteren Strukturen z.B. aus Druckmaterialien bestehen können, die mittels des zusätzlichen Drucksystems nicht verarbeitet werden können. Die universelle Verwendbarkeit der Vorrichtung wird dadurch gesteigert.

In einer weiteren vorteilhaften Aubildung können mit der Vorrichtung auch Bestandteile des dreidimensionalen Objekts z.B. durch punktuelles Auftragen geeigneter Lösungsmittel oder Säuren entfernt werden, wobei hierzu in zumindest einem Vorratsbehälter ein solches Lösungsmittel oder eine solche Säure vorrätig gehalten ist.

Bei einer weiteren vorteilhaften Ausführungsvariante weist die Dosiervorrichtung mindestens zwei Dosierkanäle auf, mittels welcher nacheinander oder gleichzeitig Druckmaterial oder Druckmaterialien aus einem oder mehreren Vorratsbehältern aufnehmbar und danach zu mindestens einer in allen drei Raumdimensionen definierten Zielposition transportierbar und dort auf das Substrat bzw. das darauf angeordnete oder darauf im Aufbau befindliche dreidimensionale Objekt aufbringbar ist bzw. sind. Der Aufbau bzw. die Bearbeitung des dreidimensionalen Objekts kann so beschleunigt werden.

Im Folgenden wird die Erfindung anhand von in den Zeichnungen dargestellten Ausführungsbeispielen detaillierter beschrieben. Es zeigen:
- Fig. 1-7 -: eine schematische Darstellung eines ersten Ausführungsbeispiels der erfindungsgemässen Vorrichtung während verschiedener Arbeitsschritte des Druckprozesses;
- Fig. 8-13 -: eine schematische Schnittansicht eines Dosierwerkzeugs während verschiedener Arbeitsschritte des Aufbaus eines dreidimensionalen Druckobj ekts;
- Fig. 14-19 -: eine schematische perspektivische Detaildarstellung des Aufbaus eines dreidimensionalen Druckobjekts während verschiedener Arbeitsschritte des Druckprozesses;
- Fig. 20 -: eine schematische Darstellung eines weiteren Ausführungsbeispiels der erfindungsgemässen Vorrichtung mit einem integrierten zusätzlichen Drucksystem;
- Fig. 21 -: eine alternative Ausbildung eines Dosierwerkzeugs;
- Fig. 22 -: eine schematische Darstellung einer Detailvariante der Vorrichtung;
- Fig. 23-28 -: eine schematische Schnittansicht eines Dosierwerkzeugs während verschiedener Arbeitsschritte des Aufbaus eines dreidimensionalen Druckobjekts mit zwei unterschiedlichen unvermischten Druckmaterialien;
- Fig. 29-34 -: eine schematische Schnittansicht eines Dosierwerkzeugs während verschiedener Arbeitsschritte des Aufbaus eines dreidimensionalen Druckobjekts mit zwei unterschiedlichen Druckmaterialien, die vermischt werden;
- Fig. 35-38 -: schematisch die Bearbeitung eines dreidimensionalen Objekts mit Druckmaterialelementen mit materialabbauender Wirkung; und
- Fig. 39 -: eine schematische perspektivische Darstellung einer Dosiervorrichtung mit vier Dosierkanälen.

Für die nachstehende Beschreibung gilt die folgende Festlegung: Sind in einer Figur zum Zweck zeichnerischer Eindeutigkeit Bezugszeichen angegeben, aber im unmittelbar zugehörigen Beschreibungsteil nicht erwähnt, so wird auf deren Erläuterung in vorangehenden oder nachfolgenden Beschreibungsteilen verwiesen. Umgekehrt sind zur Vermeidung zeichnerischer Überladung für das unmittelbare Verständnis weniger relevante Bezugszeichen nicht in allen Figuren eingetragen. Hierzu wird auf die jeweils übrigen Figuren verwiesen.

Die auf die jeweilige Unterlage (Substrat bzw. darauf angeordnetes oder im Aufbau begriffenes dreidimensionales Objekt) bei jedem Druckschritt aufgebrachten Mengeneinheiten des Druckmaterials werden unabhängig von ihrer konkreten geometrischen Form als Druckmaterialelemente bezeichnet. Unter Druckmaterialelementen werden im Zusammenhang mit der Erfindung sowohl Druckmaterialpunkte im engeren Sinn als auch linienförmige oder flächige Druckmaterialstrukturen verstanden.

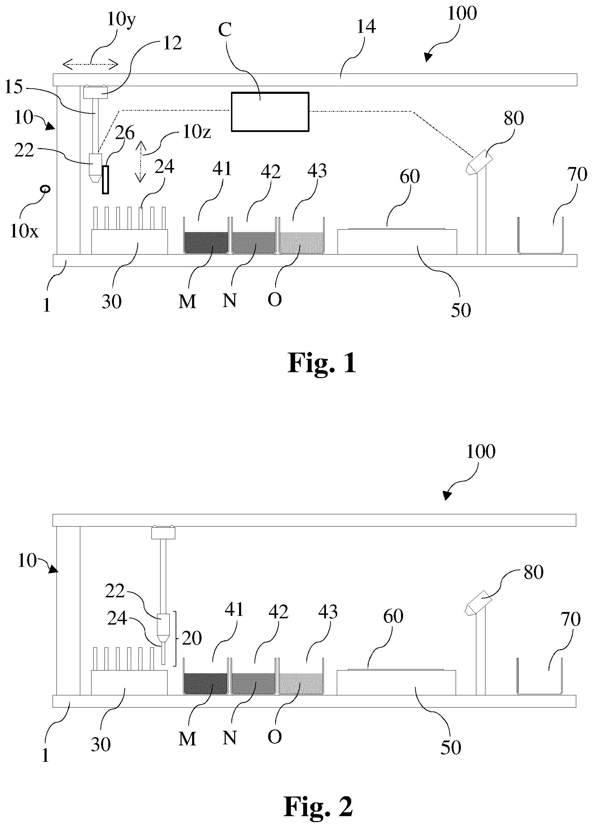

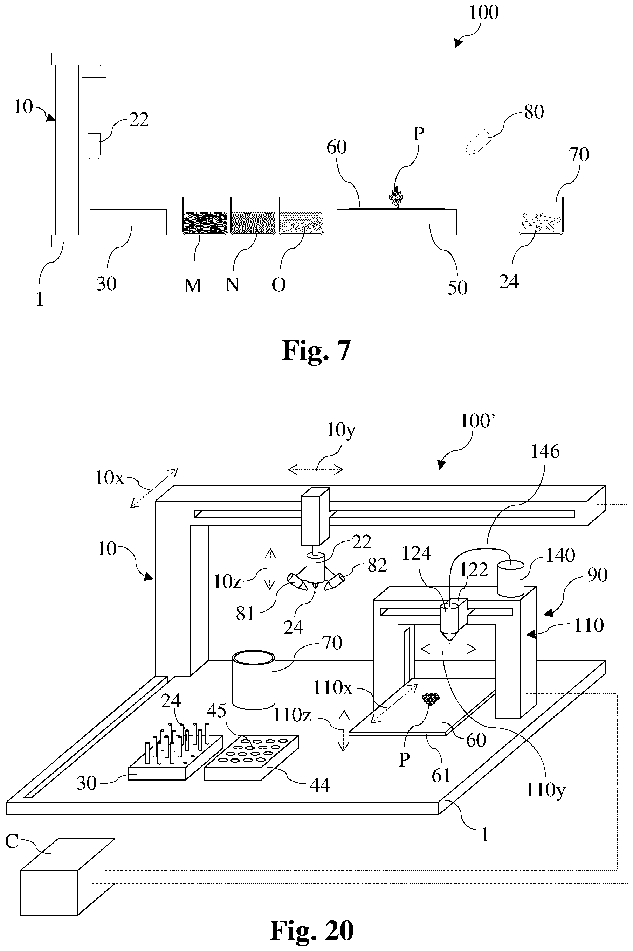

Fig. 1 zeigt ein erstes Ausführungsbeispiel der erfindungsgemässen Vorrichtung im Ruhezustand. Die als Ganze mit 100 bezeichnete Vorrichtung ist auf einer Grundplatte 1 aufgebaut, auf welcher ein Handhabungsroboter 10 angeordnet ist. Am Handhabungsroboter 10 ist ein Dosierkopf 22 montiert. Der nur schematisch dargestellte Handhabungsroboter 10 ist dazu ausgebildet, den an ihm montierten Dosierkopf 22 zu jeder beliebigen Raumkoordinate innerhalb der Vorrichtung 100 zu bewegen, und stellt somit eine Transporteinrichtung für den Dosierkopf dar. Als Ganzes ist der Handhabungsroboter 10 relativ zur Grundplatte 1 in Richtung des hier nur durch einen Kreis angedeuteten Pfeils 10x senkrecht zur Zeichenebene vor und zurück bewegbar (siehe dazu auch Fig. 20). Ein Schlitten 12 ist längs eines sich in Richtung des Pfeils 10y erstreckenden Roboterarms 14 links und rechts bewegbar angeordnet und ein am Schlitten 12 angeordneter Roboterarm 15, der den Dosierkopf 22 trägt, kann den Dosierkopf 22 in Richtung des Pfeils 10z auf und ab bewegen. Der Handhabungsroboter 10 wird von einem Steuerungsrechner C gesteuert. Der Handhabungsroboter 10 kann auch weitere Achsen (z.B. zur Rotation des Roboterarms 15) aufweisen oder er kann z.B. auch als Linear-Roboter, Gelenkarm-Roboter, SCARA-Roboter, Delta-Roboter etc. ausgebildet sein. Wesentlich ist lediglich, dass er, wie schon gesagt, den Dosierkopf 22 zu jeder beliebigen Raumkoordinate innerhalb der Vorrichtung 100 bewegen kann. Vorteilhaft ist dabei, dass der Handhabungsroboter 10 mindestens dieselbe räumliche Auflösung und Positioniergenauigkeit erlaubt wie in handelsüblichen 3D-Druckern, also mindestens 100-200 µm, vorteilhafterweise bis hinunter zu 1-2 µm. Der dargestellte Handhabungsroboter 10 entspricht dem Stand der Technik, wie er z.B. durch das Dokument WO 2016/074105 A1 illustriert ist.

Auf der Grundplatte 1 sind ein Halter 30 für Dosierwerkzeuge 24, (hier z.B. drei) Vorratsbehälter 41, 42 und 43 für hier verschiedene Druckmaterialien M, N, O, eine Analysenwaage 50 mit einem darauf eingespannten Substrat 60, ein Abfallbehälter 70 für verbrauchte Dosierwerkzeuge 24 und eine Strahlungsquelle 80 angeordnet.

Der Dosierkopf 22 ist dazu ausgebildet, mit Hilfe des Handhabungsroboters 10 Dosierwerkzeuge 24 aufzunehmen, zu bedienen, und wieder abzugeben. Der Dosierkopf 22 bildet zusammen mit einem aufgenommenen Dosierwerkzeug 24 eine Dosiervorrichtung 20. Die dargestellte Dosiervorrichtung 20 entspricht dem Stand der Technik, wie er z.B. durch die Dokumente WO 2016/074105 A1 oder WO 2017/152293 A1 illustriert ist. Der Dosierkopf 22 kann aber auch anders realisiert sein, sofern er zur Aufnahme, Bedienung und Abgabe von Dosierwerkzeugen ausgebildet ist. Die Dosiervorrichtung kann beispielsweise auch durch eine handelsübliche Dosiervorrichtung wie Liquid-Handler, Kartuschendosierer, etc. gebildet sein.

Der Handhabungsroboter 10 erlaubt es, die Dosiervorrichtung 20 in verschiedenen Raumachsen so zu bewegen, dass diese sowohl Druckmaterial-Vorratsbehälter als auch jeden gewünschten Punkt auf dem zu druckenden dreidimensionalen Objekt anfahren kann.

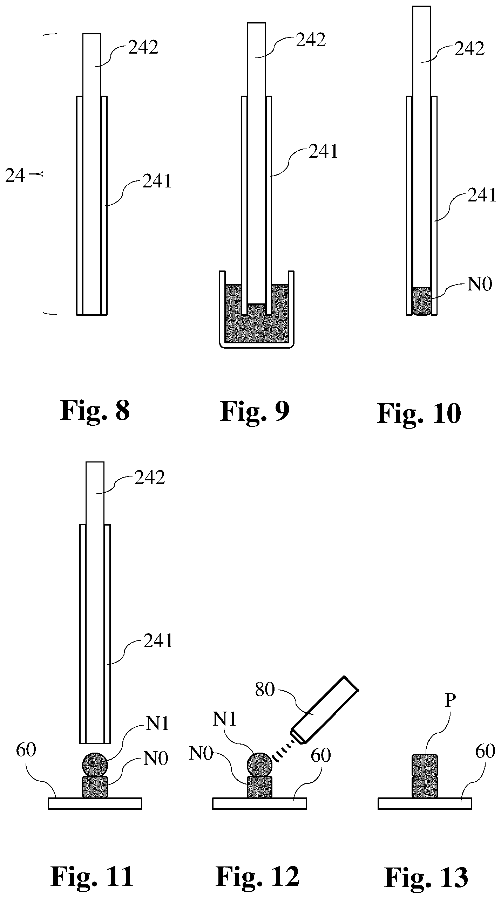

Der Halter 30 hält eine Anzahl Dosierwerkzeuge 24 vorrätig. Die Dosierwerkzeuge 24 sind dabei als Röhrchen bzw. Kapillaren 241 mit einem darin gleitenden Kolben 242 ausgebildet (siehe auch Fig. 8-11), wobei sie zum Beispiel durch Zurückziehen des Kolbens 242 eine Kavität bilden, so dass eine durch die Grösse der Kavität definierte Menge an Druckmaterial im Dosierwerkzeug 24 verbleibt. So kann etwa eine Flüssigkeit aufgesaugt werden. Ebenso eignet sich das so ausgebildete Dosierwerkzeug 24, um damit in feste, wachsartige, granuläre und pulverförmige Feststoffe einzustechen und Teilmengen davon aufzunehmen. Die genaue Funktionsweise solcher Dosierwerkzeuge ist z.B. im Dokument WO 2016/074105 A1 beschrieben.

Alternativ können die Dosierwerkzeuge auch als handelsübliche automatische Pipetten mit wegwerfbaren Spitzen oder Spritzen ausgebildet sein, welche beispielsweise nach dem Luftverdrängungspinzip (air displacement) resp. dem Direktverdrängungsprinzip (positive displacement) funktionieren. Deren Funktionsweise ist dem Fachmann geläufig und ist daher nicht weiter im Detail beschrieben.

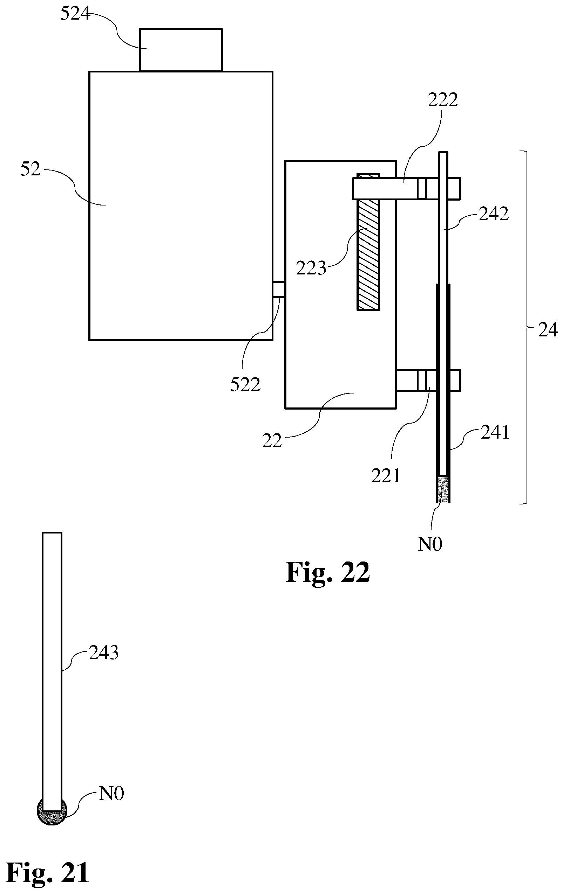

Als weitere Alternative können die Dosierwerkzeuge auch einfach als nicht-hohle Festkörper ohne Kavität ausgebildet sein, z.B. in Form dünner Stäbchen, denen beim Eintauchen/Einstechen in das Druckmaterial eine kleine Substanzmenge anhaften bleibt, die dann auf dem dreidimensionalen Objekt abgegeben werden kann (z.B. indem der Substanzpunkt aufgetupft wird, siehe auch Fig. 21). Die genaue Funktionsweise solcher Dosierwerkzeuge ist z.B. im Dokument WO 2017/152293 A1 beschrieben.

Die Vorratsbehälter 41, 42 und 43 halten drei bzw. mehrere unterschiedliche Druckmaterialien M, N und O vorrätig. Alternativ kann die Bereitstellung der Druckmaterialien auch in anderen Gefässarten erfolgen, z.B. in Platten mit mehreren Vertiefungen (z.B. sog. Microtiter-Platten, MTP), in welchen kleine DruckmaterialMengen vorrätig gelagert werden (siehe auch Fig. 20).

In einer vorteilhaften Ausführungsvariante umfasst der in den Vorratsbehältern bereitgestellte Vorrat an Druckmaterialien Materialien unterschiedlichster Art gleichzeitig, zum Beispiel verschiedene Flüssigkeiten, Feststoffe (z.B. wachsartig, granular oder pulverförmig), aber auch gefrorene Flüssigkeiten ("Cryo 3D-Printing"), Lösungen und Suspensionen, bis hin zu ganzen Zellkulturen mit lebenden Zellen, die in geeigneten Gefässen vorrätig gelagert werden. Dies erlaubt, dass für jeden definierten Punkt des dreidimensionalen Objekts genau definiert werden kann, welches der vorrätigen Materialien verwendet werden kann, was weiter erlaubt, Objekte mit grösserer Materialvielfalt und -komplexität herzustellen als bisher möglich. Neben der Verwendung von Druckmaterialien, aus denen ein dreidimensionales Objekt additiv aufgebaut wird, können natürlich auch Materialien zum Einsatz kommen, die bestehende Strukturen/Objekte punktuell entfernen, z.B. durch Wegätzen/Weglösen mit Säuren resp. Lösungsmitteln.

Es ist auch möglich, in den Vorratsbehältern 41, 42 und 43 nur jeweils kleinere Mengen an Druckmaterialien vorrätig zu halten. Die Be- bzw. gegebenenfalls Nachfüllung dieser Vorratsbehälter kann dann entweder mittels der Dosiervorrichtung 20 oder mittels einer separaten Dosiervorrichtung aus entsprechenden Quellen erfolgen. Diese Variante ist zum Beispiel besonders vorteilshaft, wenn einzelne der verwendeten Druckmaterialien in einer anderen, der Druckvorrichtung räumlich nahen Vorrichtung (z.B. einer Synthese- oder Formuliervorrichtung) hergestellt werden und zum Test in der Druckvorrichtung verwendet werden sollen. Als Vorratsbehälter würde in diesem Fall vorteilhafterweise eine Vorrichtung mit kleinen Vertiefungen (z.B. eine Mikrotiterplatte) eingesetzt werden (siehe auch Fig. 20).

Eine alternative Möglichkeit, welche nicht den vorliegenden Ansprüchen entspricht, besteht darin, die Druckmaterialien nicht vorrätig zu halten, sondern direkt auf der Vorrichtung durch geeignete Werkzeuge und Methoden herzustellen. Dies kann speziell von Wichtigkeit sein, wenn das Druckmaterial selbst schnell aushärtet, nachdem es synthetisiert oder formuliert wurde (z.B. Zweikomponenten-Kleber etc.).

Das auf der Analysenwaage 50 eingespannte Substrat 60 dient als Untergrund für das aufzubauende dreidimensionale Objekt P.

Die Strahlungsquelle 80, die z.B. als Laser oder UV-Strahlenquelle ausgebildet ist, dient dazu, um einzelne Druckmaterialelemente (Details siehe unten) miteinander zu verschmelzen oder zu härten bzw. zu vernetzen. Die Strahlungsquelle kann dabei vorteilhafterweise so in der Druckvorrichtung angebracht und (vom Steuerungsrechner C) steuerbar sein, dass sie jeden beliebigen möglichen Punkt im Raum über dem Substrat gezielt bestrahlen kann, um das an diesem Punkt befindliche Material zu (ver)schmelzen, zu härten oder zu vernetzen. In einer anderen vorteilhaften Ausbildungvariante ist die Strahlungsquelle direkt am Dosierkopf 22 angebracht und so ausgerichtet, dass sie immer genau auf die Spitze des in den Dosierkopf 22 eingesetzten Dosierwerkzeugs 24 zielt, so dass ein dort abgegebenes Druckmaterialelement unmittelbar nach dessen Abgabe verschmolzen, gehärtet oder vernetzt werden kann (siehe auch Fig. 20).

Die Analysenwaage 50 dient dazu, die von der Dosiervorrichtung 20 abgegebene Menge an Druckmaterial gravimetrisch zu messen. Vorteilhafterweise kann alternativ oder zusätzlich mittels einer weiteren Analysenwaage auch die von der Dosiervorrichtung aufgenommene Menge an Druckmaterial gravimetrisch gemessen werden. Eine Möglichkeit dazu besteht darin, dass die Vorratsbehälter auf einer Analysenwaage angeordnet sind, so dass die aus ihnen entnommenen Druckmaterialmengen bestimmt werden können. Eine besonders geeignete Anordnung zur Bestimmung der von der Dosiervorrichtung bzw. deren Dosierwerkzeug aufgenommenen Druckmaterialmengen ist in Fig. 22 schematisch dargestellt.

Bei dieser Anordnung ist der Dosierkopf 22 über ein Verbindungsstück 522 an einer Analysenwaage 52 aufgehängt, so dass die Analysenwaage 52 das Gewicht des Dosierkopfs 22 mit allen an ihm befestigten Objekten, insbesondere einem Dosierwerkzeug 24, misst. Die Analysenwaage 52 ist über ein Verbindungsstück 524 mit dem hier nicht dargestellten Handhabungsroboter 10 verbunden. Wie aus Fig. 22 weiter erkennbar ist, ist der Dosierkopf 22 mit zwei Greifzangenpaaren 221 und 222 ausgestattet. Das in der Fig. 22 untere Greifzangenpaar 221 ist bezüglich des Dosierkopfs 22 stationär und dient zum Festhalten/Freigeben eines Dosierwerkzeugs 24 bzw. hier dessen Röhrchens 241. Das andere Greifzangenpaar 222 ist über einen Antrieb 223 auf- und ab verstellbar und dient zum Festhalten/Loslassen des Kolbens 242 des Dosierwerkzeugs 24 sowie zum Bewegen desselben aufwärts und abwärts. Die beiden Greifzangenpaare 221 und 222 und der Antrieb 223 sind ebenfalls vom (hier nicht dargestellten) Steuerungsrechner C gesteuert. Eine detailliertere Erläuterung findet sich zum Beispiel im Dokument WO 2016/074105 A1.

Mit der Analysenwaage 52 kann das Gewicht eines eingespannten Dosierwerkzeugs 24 mit bzw. ohne aufgenommenes Druckmaterial vor und nach der Druckmaterialaufnahme und auch nach der Druckmaterialabgabe gemessen werden. Auf diese Weise kann die Menge des auf das dreidimensionale Objekt aufzutragenden Druckmaterialelements genau bestimmt werden. Anhand der gemessenen Materialmenge kann auch eine Entscheidung gefällt werden, ob das betreffende Druckmaterialelement überhaupt aufgetragen oder verworfen werden soll. Falls die Menge zu gering ist, kann das Dosierwerkzeug beispielsweise noch einmal in den entsprechenden Vorratsbehälter eingestochen bzw. eingetaucht werden, um zusätzliches Druckmaterial aufzunehmen. Falls die Menge zu gross ist, kann das Dosierwerkzeug verworfen und neues Druckmaterial mit einem anderen, evtl. kleineren Dosierwerkzeug aufgenommen werden.

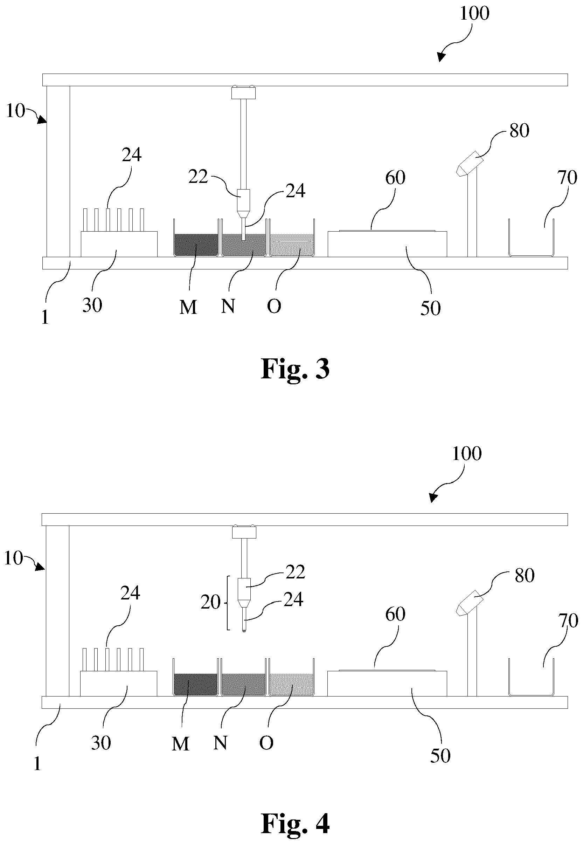

Fig. 2 zeigt einen ersten Arbeitsschritt der erfindungsgemässen Druckvorrichtung 100. Der Dosierkopf 22 wird vom Handhabungsroboter 10 zum Halter 30 gefahren, wo er ein Dosierwerkzeug 24 aufnimmt, welches hier z.B. aus einem Röhrchen 241 und einem darin verschiebbar angeordneten Kolben 242 besteht. In einem nächsten Schritt (Fig. 3) wird das Dosierwerkzeug 24 in ein im Vorratsbehälter 42 gelagertes Druckmaterial N gestochen, falls es sich um einen Feststoff, eine hochviskose Flüssigkeit oder Suspension handelt, bzw. getaucht, falls es sich um eine Flüssigkeit handelt. Im Fall einer Flüssigkeit oder Suspension wird der Kolben 242 (Fig. 8-11) des Dosierwerkzeugs 24 danach zurückgezogen, um eine definierte Menge an Druckmaterial N aufzunehmen (siehe auch Fig. 9), so dass die Menge N0 (siehe Fig. 10) in der zwischen Kolben 242 und Röhrchen 241 geformten Kavität verbleibt. Die genaue Menge N0 kann durch die Distanz, die der Kolben 242 zurückgezogen wird und den Innendurchmesser des Röhrchens 241 gesteuert werden. Üblicherweise eignen sich Röhrchen mit einem Innendurchmesser von 0.1 bis 1 mm, je nach Art des zu verarbeitenden Druckmaterials.

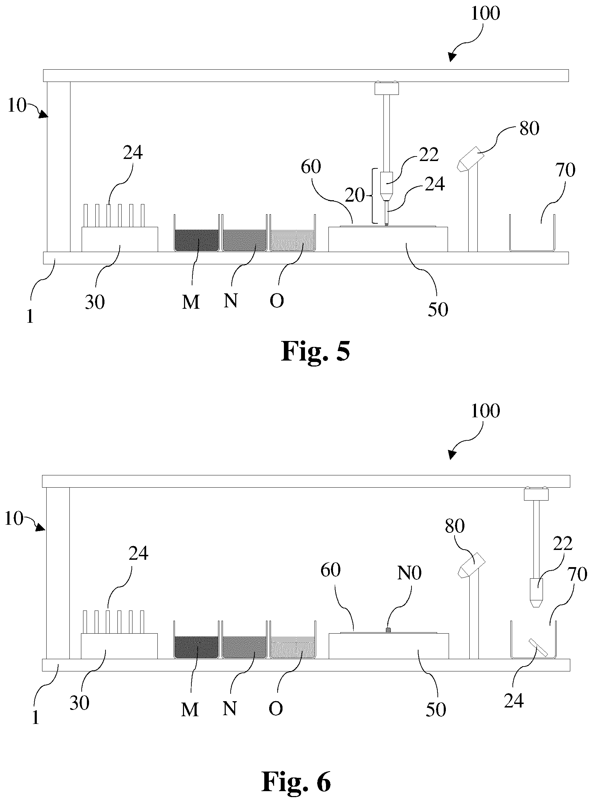

Nach Aufnehmen/Aufsaugen der Druckmaterialmenge N0 in das Dosierwerkzeug 24 wird dieses wieder aus dem Druckmaterial N gehoben (Fig. 4), und anschliessend zum Substrat 60 bewegt, wo die Druckmaterialmenge N0 als erstes Druckmaterialelement des herzustellenden dreidimensionalen Objekts P an einer genau definierten Stelle auf dem Substrat S aufgebracht wird (Fig. 5). Dazu wird die Materialmenge N0 durch Herunterbewegen des Kolbens 242 innerhalb des Dosierwerkzeugs 24 mittels des Dosierkopfs 22 aus dem Dosierwerkzeug 24 ausgestossen und auf das Substrat S aufgebracht. Natürlich können auch gleichzeitig mehrere Portionen eines gleichen Druckmaterials von einem Dosierwerkzeug aufgenommen werden, die dann nacheinander und unabhängig voneinander auf dem dreidimensionalen Objekt P platziert werden können. Durch Bestrahlung bzw. Hitzeeinwirkung mittels der Strahlungsquelle 80 (z.B. eines für das entsprechende Druckmaterial geeigneten Lasers) wird das erste Druckmaterialelement N0 gehärtet bzw. vernetzt (Fig. 6).

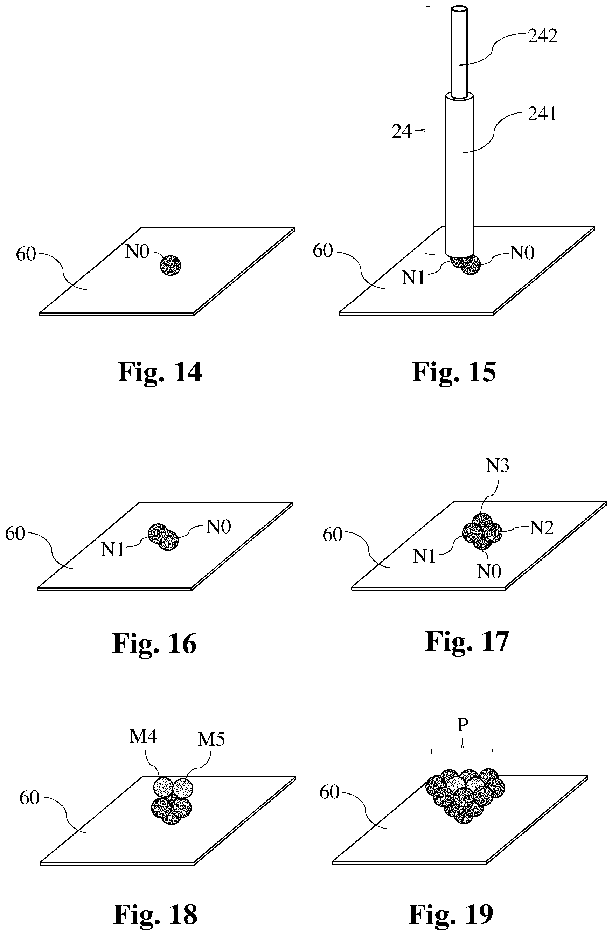

Falls nun weitere Druckmaterialelemente desselben Typs zum Aufbau des Objekts P verwendet werden sollen, so wird der Eintauch-/Aufnahme-/Abgabe-Ablauf wie in Figuren 3-6 beschrieben wiederholt, um Druckmaterialelement an Druckmaterialelement zu setzen (siehe auch Fig. 14-17), um ein dreidimensionales Objekt P zu erstellen. Bei ausreichender Druckmaterialaufnahme können allenfalls auch mehrere Druckmaterialelemente nacheinander aufgebracht werden, ohne dass das Dosierwerkzeug 24 dazwischen in den Vorratsbehälter eingetaucht werden muss. Soll aber ein anderes Druckmaterial (hier z.B. M oder O) verwendet werden, so wird das verbrauchte bzw. kontaminierte Dosierwerkzeug 24, mit dem das Material N dosiert wurde, in den Abfallbehälter 70 weggeworfen (Fig. 6), und der Druckvorgang analog den in den Fig. 2-5 beschriebenen Schritten mit einem anderen Druckmaterial solange wiederholt, bis schliesslich das aus einer Vielzahl einzelner, miteinander verschmolzener resp. gehärteter Druckmaterialelemente bestehende Endprodukt als dreidimensionales Objekt P fertiggestellt ist (Fig. 7). Alternativ könnten verwendete verschmutzte Dosierwerkzeuge vor nächsten Dosierschritten auch nicht verworfen, sondern einfach mittels einer geeigneten, vorrichtungsinternen Waschvorrichtung gereinigt werden.

Die Fig. 8-13 zeigen Detailschritte des erfindungsgemässen Druckverfahrens. Fig. 8 zeigt ein Dosierwerkzeug 24, hier in einer einfachen Form bestehend aus einem Röhrchen 241 aus Glas (geeignet sind aber je nach zu verwendenden Druckmaterialien auch andere Werkstoffe), in dem ein etwas längerer Kolben 242 (vorteilhafterweise ebenfalls aus Glas) gleitend gelagert ist. Der Kolben 242 kann mittels des Dosierkopfs 22 bzw. des Handhabungsroboters 10 relativ zum Röhrchen 241 auf und ab verstellt werden.

Fig. 9 zeigt, wie das Dosierwerkzeug 24 in ein - im Darstellungsbeispiel flüssiges - Druckmaterial N getaucht wird, welches durch Zurückziehen des Kolbens 242 in das Röhrchen 241 gesaugt wird. Im Falle eines Feststoffs (z.B. Pulver, Granulat oder amorpher Feststoff) würde der Kolben schon vor dem Einstechen in den Feststoff auf eine definierte Distanz zurückgezogen (um eine Kavität definierter Grösse zu erhalten) und das Werkzeug dann in den Feststoff gesteckt, wobei ein Teil des Feststoffs in das Röhrchen 241 gelangen und dort verbleiben würde.

Fig. 10 zeigt ein vollständig mit einer definierten Druckmaterialmenge N0 gefülltes Dosierwerkzeug 24, wie es zum Beispiel während der Bewegung der Dosiervorrichtung 20 vom Vorratsbehälter 42 zum Substrat 60 der Fall ist.

Fig. 11 stellt dar, wie eine im Dosierwerkzeug 24 transportierte Druckmaterialmenge N1 zu seiner Zielposition (hier direkt auf einem bereits vorher auf dem Substrat 60 platzierten und gehärteten Druckmaterialelement N0) gebracht, genau positioniert, und durch Herunterfahren des Kolbens 242 aus dem Dosierwerkzeug 24 gedrückt und auf das Druckmaterialelement N0 aufgebracht wird.

Wie in Fig. 12 dargestellt, wird das soeben aufgebrachte Druckmaterialelement N1 durch Einwirken von mittels der Strahlungsquelle 80 (je nach Druckmaterial) erzeugter Hitze / Strahlung / Laserstrahl mit dem bereits abgelegten Druckmaterialelement N0 verschmolzen oder gehärtet bzw. vernetzt, so dass sich aus den beiden Druckmaterialelementen ein dreidimensionales Objekt P bildet (Fig. 13). Sollten zur Herstellung des dreidimensionalen Objekts Druckmaterialien mit unterschiedlichen Schmelz-/Härtungs-/Vernetzungsverfahren benötigt werden, so ist die erfindungsgemässe Druckvorrichtung selbstverständlich auch mit mehreren, jeweils für die unterschiedlichen Druckmaterialien geeigneten Schmelz/Härtungs-/Vernetzungsvorrichtungen ausgestattet (siehe auch Fig. 20). Je nach Druckmaterial kann auch komplett auf Härten oder Schmelzen oder Vernetzen und dementsprechende Vorrichtungen verzichtet werden, dies etwa bei Druckmaterialien, die ohne Fremdeinwirkung aushärten bzw. erstarren (z.B. selbsttrocknende Materialien, Zweikomponenten-Systeme, ...).

Fig. 21 zeigt ein alternatives, stabförmiges Dosierwerkzeug 243, welches einfach in das Druckmaterial (hier N) eintauchbar ist, wobei an ihm eine Druckmaterialmenge N0 haften bleibt, die dann auf dem Objekt P platziert werden kann.

Die Bewegungen des Dosierkopfs 22 mittels des Handhabungsroboters 10 in die drei Raumrichtungen zur Aufnahme bzw. zum Verwerfen eines Dosierwerkzeugs 24, zum Eintauchen und zur Entnahme des Dosierwerkzeugs 24 in ein bzw. aus einem Druckmaterial und zur Positionierung des Dosierwerkzeugs am gewünschten Ablageort auf dem Substrat 60 bzw. dem darauf in Aufbau befindlichen dreidimensionalen Objekt P werden wie bei dem in Fig. 20 dargestellten Ausführungsbeispiel vom Steuerungsrechner C gesteuert, ebenso das Einsaugen von Druckmaterial in das Dosierwerkzeug 24 und das Ausstossen desselben aus dem Dosierwerkzeug und die Aktivierung der Strahlungsquelle 80.

Der Steuerungsrechner verwendet hierfür Prozesssteuerungsinformationen, die für jeden zu druckenden Einzelpunkt (oder Linie, falls das Dosierwerkzeug während der Abgabe in einer oder mehreren Raumachsen bewegt wird) dessen Raumkoordinaten, Materialidentität und Materialmenge, sowie die Druckreihenfolge aller Einzelpunkte des dreidimensionalen Objekts umfassen.

Die Fig. 14-19 zeigen den eigentlichen Druckvorgang stark vereinfacht in perspektivischer Darstellung, um den Aufbau eines dreidimensionalen Objekts P aus einzelnen Druckmaterialelementen zu erklären. Fig. 14 zeigt ein Substrat 60 mit einem ersten darauf bereits aufgebrachten Druckmaterialelement N0. In Fig. 15 ist dargestellt, wie mittels des Dosierwerkzeugs 24 ein zweites Druckmaterialelement N1 auf das Druckmaterialelement N0 aufgebracht wird (auf die Darstellung des Schmelz-/Härtungsvorgangs ist in den Fig. 14-18 der Einfachheit halber verzichtet), so dass sich, wie in Fig. 16 dargestellt, ein einfaches Objekt aus zwei Druckmaterialelementen bildet.

Fig. 17 zeigt das im Aufbau befindliche Objekt mit zwei weiteren Druckmaterialelementen N2 und N3, die in diesem Beispiel insgesamt einen einfachen, auf dem Kopf stehenden Tetraeder bilden. Fig. 18 zeigt nun, wie dem Objekt zwei weitere Druckmaterialelemente M4 und M5, dieses Mal aber aus einem anderen Druckmaterial M, hinzugefügt wurden. Fig. 19 schliesslich zeigt einen weiteren späteren Fertigungsschritt, bei dem dem Objekt P bereits weitere Druckmaterialelemente aus Material N hinzugefügt wurden. Es lässt sich so einfach sehen, wie ein aus verschiedenen Druckmaterialien bestehendes dreidimensionales Druckobjekt P mittels der erfindungsgemässen Druckvorrichtung 100 aufgebaut werden kann.

Die erfindungsgemässe Vorrichtung kann auch eine Heizung 26 (nur in Fig. 1 schematisch dargestellt) aufweisen, um die im Dosierwerkzeug 24 enthaltene Menge des Druckmaterials z.B. in geschmolzenem Zustand zu halten, während sie zum Auftragungspunkt transportiert wird.

Ein zweites Ausführungsbeispiel der erfindungsgemässen Vorrichtung zur Herstellung eines dreidimensionalen Objekts ist schematisch und stark vereinfacht in Fig. 20 dargestellt. Die Vorrichtung 100' umfasst, wie bereits vorgängig erläutert, wieder einen Handhabungsroboter 10, welcher einen Dosierkopf 22 in alle drei Raumrichtungen (Pfeile 10x, 10y und 10z) bewegen kann, um so mittels des Dosierkopfs 22 ein Dosierwerkzeug 24 aus einem Halter 30 aufzunehmen, damit Druckmaterial aus einem Vorratsbehälter (hier in Form einer Platte 44 mit mehreren verschiedene Druckmaterialien enthaltenden Vertiefungen 45) aufzunehmen, damit das dreidimensionale Objekt P aufzubauen und verbrauchtes Dosierwerkzeug 24 in einen Abfallbehälter 70 zu verwerfen.

Bei diesem Ausführungsbeispiel sind mehrere (hier konkret zwei) Strahlungsquellen 81 und 82 direkt am Dosierkopf 22 angebracht und so ausgerichtet, dass sie auf die Spitze des im Dosierkopf 22 eingesetzten Dosierwerkzeugs 24 zielen. Dies erlaubt, dass in der Vorrichtung mehrere unterschiedliche Druckmaterialien mit unterschiedlichen Ansprüchen an die Strahlungsquelle zur Härtung oder Schmelzung eingesetzt werden können, ohne die Vorrichtung als Ganze zu verändern.

Ein Steuerungsrechner C steuert wiederum den Handhabungsroboter 10 und die hier zwei Strahlungsquellen 81 und 82 sowie den Dosierkopf 22 in der schon im Zusammenhang mit dem ersten Ausführungsbeispiel erläuterten Weise.

Zusätzlich ist bei diesem zweiten Ausführungsbeispiel in die Druckvorrichtung 100' noch ein eigenständiges Drucksystem 90 integriert, das für sich ebenfalls zur Herstellung eines dreidimensionalen Objekts ausgebildet ist. Das zusätzliche Drucksystem 90 kann beispielsweise durch einen herkömmlichen 3D-Drucker gebildet sein.

Im dargestellten Beispiel umfasst das zusätzliche Drucksystem 90 eine als Ganzes mit 110 bezeichnete Antriebseinrichtung, mittels welcher einerseits ein plattenförmiger Substratträger 61, auf dem ein Substrat 60 befestigt ist, in seiner Ebene nach vor und nach hinten (Pfeil 110x) und senkrecht zu seiner Ebene nach oben und nach unten (Pfeil 110z) verstellt werden kann und anderseits ein Druckkopf 122, an dem eine Dosiervorrichtung 124 montiert ist, parallel zur Ebene des Substratträgers 61 nach links und rechts (Pfeil 110y) bewegt werden kann. Insgesamt kann somit die Dosiervorrichtung 124 in jede beliebige Raumposition relativ zum Substratträger 61 bzw. zum darauf befestigten Substrat 60 bewegt werden. Der Dosiervorrichtung 124 wird aus einem Vorratsbehälter 140 über eine Leitung 146 ein z.B. flüssiges Druckmaterial zugeführt und die Dosiervorrichtung 124 ist dazu ausgebildet, Druckmaterial punktweise abzugeben, um daraus auf dem Substrat 60 ein dreidimensionales Objekt P aufzubauen. Das zusätzliche Drucksystem 90 ist, wie schon erwähnt, durch einen herkömmlichen 3D-Drucker gebildet und bedarf daher keiner näheren Erläuterung. Für das zusätzliche Drucksystem 90 können grundsätzlich viele unterschiedliche 3D-Druckertypen und -technologien zum Einsatz kommen, z.B. auch der im Dokument NL 2 017 088 A beschriebene 3D-Drucker.

Der Handhabungsroboter 10 kann die an ihm befestigte, aus Dosierkopf 22 und Dosierwerkzeug 24 bestehende Dosiervorrichtung 20 ebenfalls in jede beliebige Raumposition relativ zum Substratträger 61 bzw. dem auf diesem befestigten Substrat 60 bewegen, so dass auch mittels der Dosiervorrichtung 20, wie schon oben erläutert, ein dreidimensionales Objekt P auf dem Substrat 60 aufgebaut werden kann. Somit sind nun sowohl die Dosiervorrichtung 20 als auch die Dosiervorrichtung 124 des integrierten Drucksystems 90 dazu in der Lage, am selben auf dem Substrat 60 im Aufbau befindlichen Objekt P zu arbeiten. Der Steuercomputer C übernimmt dabei die Steuerung des zusätzlichen Drucksystems 90 sowie die Koordination der Abläufe, um einerseits Kollisionen zu verhindern und andererseits den optimalen Aufbau des Objekts sicherzustellen.

Dieses zweite Ausführungsbeispiel der erfindungsgemässen Vorrichtung hat den Vorteil, dass sie universeller einsetzbar ist. So kann entweder mittels der Dosiervorrichtung 20 oder mittels des integrierten Drucksystems 90 oder vorteilhafterweise kombiniert mittels beider ein dreidimensionales Objekt aufgebaut werden. So könnte zum Beispiel das integrierte Drucksystem 90 die Grundstruktur des herzustellenden Objekts aufbauen, während mittels der Dosiervorrichtung 20 weitere Strukturen aus vom integrierten Drucksystem 90 nicht verarbeitbaren Druckmaterialien hinzugefügt werden könnten. Um die Einsatzmöglichkeiten der erfindungsgemässen Vorrichtung noch weiter zu erhöhen, kann die Vorrichtung ferner auch mit zwei oder mehreren zusätzlichen Drucksystemen ausgestattet sein.

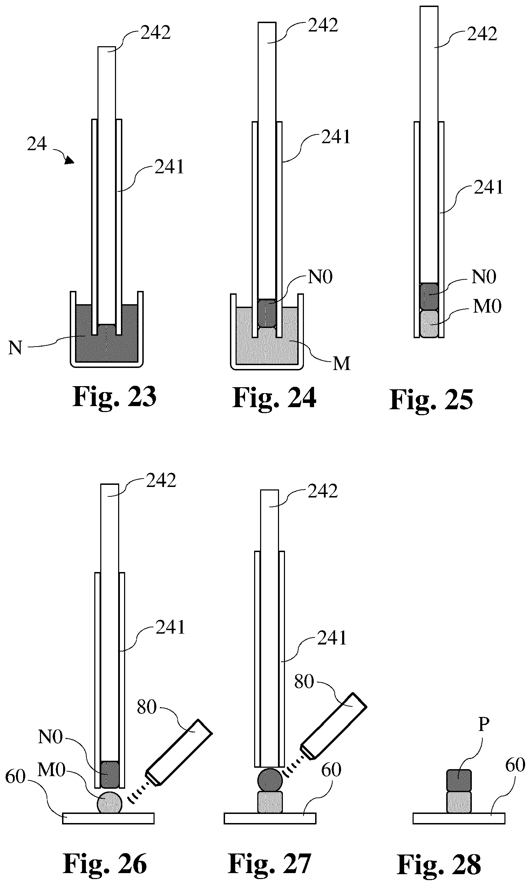

Die Fig. 23-28 zeigen Detailschritte einer Ausführungsvariante des erfindungsgemässen Druckverfahrens zum beschleunigten Aufbauen eines dreidimensionalen Objekts mit zwei unterschiedlichen unvermischten Druckmaterialien N und M. Fig. 23 zeigt wiederum das Dosierwerkzeug 24 mit dem Röhrchen 241 und dem Kolben 242, welcher mittels des Dosierkopfs 22 bzw. des Handhabungsroboters 10 relativ zum Röhrchen 241 auf und ab verstellt werden kann. Das Dosierwerkzeug 24 ist in ein Druckmaterial N getaucht, welches durch Zurückziehen des Kolbens 242 in das Röhrchen 241 gesaugt wird.

Fig. 24 zeigt das mit einer definierten Druckmaterialmenge N0 gefüllte Dosierwerkzeug 24 nach dem Herausziehen aus dem Druckmaterial N und dem Eintauchen in das Druckmaterial M. Durch weiteres Zurückziehen des Kolbens 242 wird Druckmaterial M in das Röhrchen 241 gesaugt.

Fig. 25 zeigt das mit der definierten Druckmaterialmenge N0 und der definierten Druckmaterialmenge M0 gefüllte Dosierwerkzeug 24, wobei die beiden unterschiedlichen Druckmaterialien nicht vermischt sind. Das Dosierwerkzeug 24 wird so zum Substrat 60 bewegt.

Die Fig. 26 und 27 stellen dar, wie die Druckmaterialmenge M0 und die Druckmaterialmenge N0 in ihrer Zielposition auf dem Substrat 60 bzw. dem Druckmaterialelement M0 genau positioniert und durch Herunterfahren des Kolbens 242 aus dem Dosierwerkzeug 24 gedrückt und aufgebracht werden. Durch Einwirken der Strahlungsquelle 80 erfolgt eine Verschmelzung oder Härtung bzw. Vernetzung, so dass sich aus den beiden Druckmaterialelementen M0 und N0 ein dreidimensionales Objekt P bildet (Fig. 28).

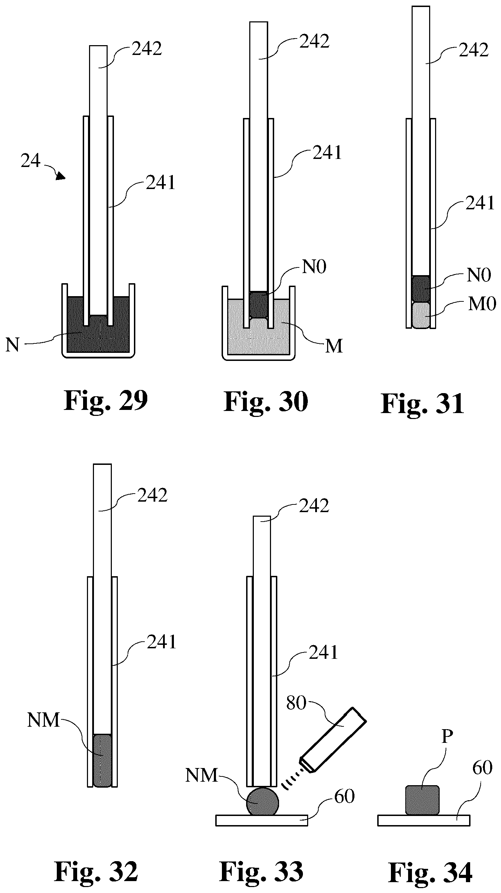

Die Fig. 29-34 zeigen Detailschritte einer Ausführungsvariante des erfindungsgemässen Druckverfahrens zum beschleunigten Aufbauen eines dreidimensionalen Objekts mit zwei unterschiedlichen Druckmaterialien N und M, die vermischt werden. Fig. 29 zeigt wiederum das Dosierwerkzeug 24 mit dem Röhrchen 241 und dem Kolben 242, welcher mittels des Dosierkopfs 22 bzw. des Handhabungsroboters 10 relativ zum Röhrchen 241 auf und ab verstellt werden kann. Das Dosierwerkzeug 24 ist in ein Druckmaterial N getaucht, welches durch Zurückziehen des Kolbens 242 in das Röhrchen 241 gesaugt wird.

Fig. 30 zeigt das mit einer definierten Druckmaterialmenge N0 gefüllte Dosierwerkzeug 24 nach dem Herausziehen aus dem Druckmaterial N und dem Eintauchen in das Druckmaterial M. Durch weiteres Zurückziehen des Kolbens 242 wird Druckmaterial M in das Röhrchen 241 gesaugt.

Fig. 31 zeigt das mit der definierten Druckmaterialmenge N0 und der definierten Druckmaterialmenge M0 gefüllte Dosierwerkzeug 24, wobei die beiden unterschiedlichen Druckmaterialien noch nicht vermischt sind. Beispielsweise durch Erzeugen von Turbulenzen durch auf und ab Verstellen des Kolbens 242 können die beiden Druckmaterialmengen N0 und M0 im Dosierwerkzeug 24 bzw. im Röhrchen 241 miteinander vermischt werden, so dass das Gemisch NM entsteht, siehe Fig. 32. Das Dosierwerkzeug 24 wird vor während oder nach dem Mischen zum Substrat 60 bewegt.

Fig. 33 stellt dar, wie das Gemisch NM als Druckmaterialelement in seiner Zielposition auf dem Substrat 60 genau positioniert und durch Herunterfahren des Kolbens 242 aus dem Dosierwerkzeug 24 gedrückt und aufgebracht wird. Durch Einwirken der Strahlungsquelle 80 erfolgt eine Verschmelzung oder Härtung bzw. Vernetzung, so dass sich das dreidimensionale Objekt P bildet (Fig. 34).

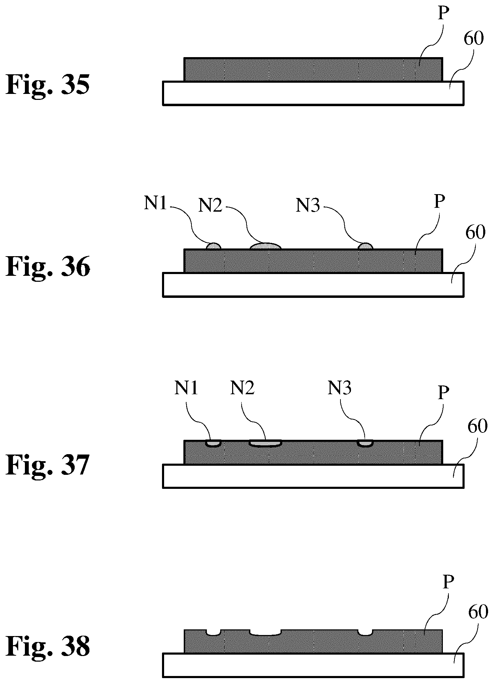

Die Fig. 35-38 zeigen die Bearbeitung eines dreidimensionalen Objekts P auf einem Substrat 60 mit Druckmaterialelementen mit materialabbauender Wirkung. Fig. 35 zeigt das noch unbearbeitete dreidimensionale Objekt P auf dem Substrat 60.

Fig. 36 stellt die Situation unmittelbar nach dem Aufbringen von drei Druckmaterialelementen N1, N2 und N3 auf das dreidimensionale Objekt P dar. In Fig. 37 haben sich die Druckmaterialelemente N1, N2 und N3 bereits in das dreidimensionale Objekt P hineingefressen bzw. das entsprechende Material des Objekts P abgebaut.

Fig. 38 zeigt die Situation nach dem Entfernen, beispielsweise Herauswaschen, der Druckmaterialelemente N1, N2 und N3.

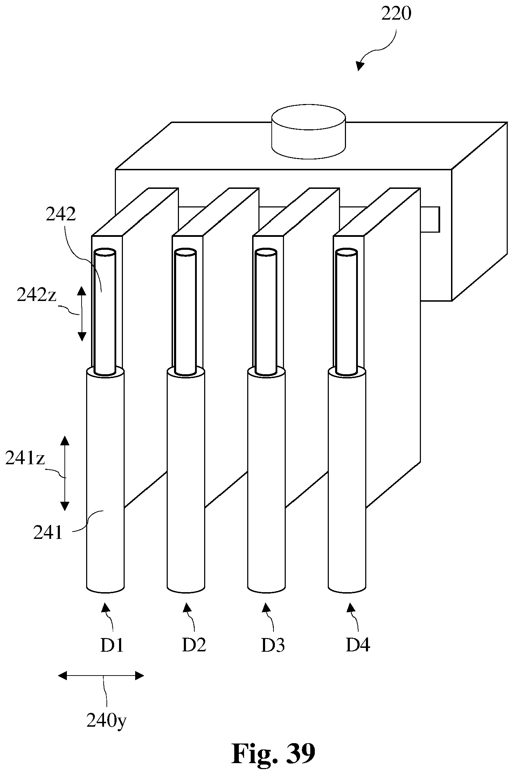

Fig. 39 zeigt eine schematische perspektivische Darstellung einer Dosiervorrichtung 220 mit vier Dosierkanälen D1, D2, D3 und D4. Jeder dieser vier Dosierkanäle D1, D2, D3 und D4 umfasst beispielsweise jeweils ein Röhrchen 241 und einen Kolben 242, wie sie zuvor beschrieben worden sind. Das Röhrchen 241 und der Kolben 242 sind jeweils auf- und ab verstellbar, wie mit Pfeilen 241z bzw. 242z dargestellt und im Zusammenhang mit Fig. 22 im Detail erläutert. Entsprechend dem Pfeil 240y sind die vier Dosierkanäle D1, D2, D3 und D4 horizontal gegeneinander verschiebbar. Mittels der vier Dosierkanäle D1, D2, D3 und D4 können nacheinander oder gleichzeitig Druckmaterial oder Druckmaterialien aus einem oder mehreren Vorratsbehältern aufgenommen und danach mittels Verstellung der Dosiervorrichtung 220 zu einer oder mehreren in allen drei Raumdimensionen definierten Zielpositionen transportiert werden. Dort kann das Druckmaterial bzw. können die Druckmaterialien auf das Substrat 60 bzw. das darauf angeordnete oder darauf im Aufbau befindliche dreidimensionale Objekt P aufgebracht werden.

## Patentansprüche

1. Verfahren zur Herstellung und/oder Bearbeitung eines dreidimensionalen Druckobjekts (P) mit einem Druckmaterial (M, N, O), das an einer Zielposition in Form von diskreten dreidimensionalen Druckmaterialelementen (N0, N1, N2, N3, M4, M5, NM) abgegeben wird, wobei eine Dosiervorrichtung (20; 220) zu mindestens einem Vorratsbehälter (41, 42, 43; 44, 45), in welchem ein Druckmaterial (M, N, O) vorrätig gehalten ist, bewegt wird, wobei mittels der Dosiervorrichtung (20; 220) aus diesem mindestens einen Vorratsbehälter (41, 42, 43; 44, 45) Druckmaterial aufgenommen wird, wobei die Dosiervorrichtung (20; 220) zu einer in allen drei Raumdimensionen definierten Zielposition bewegt wird und wobei an dieser Zielposition mittels der Dosiervorrichtung (20; 220) eine dosierte Menge Druckmaterial auf ein Substrat (60) bzw. ein darauf angeordnetes oder darauf im Aufbau befindliches dreidimensionales Druckobjekt (P) aufgebracht wird, um ein Druckmaterialelement (N0, N1, N2, N3, M4, M5, NM) zu bilden, wobei das Bilden eines Druckmaterialelements (N0, N1, N2, N3, M4, M5, NM) solange wiederholt wird, bis das dreidimensionale Druckobjekt (P) vollständig aufgebaut und/oder bearbeitet ist.

2. Verfahren nach Anspruch 1, **dadurch gekennzeichnet, dass** zwei oder mehrere unterschiedliche Druckmaterialien (M, N, O) vorrätig gehalten werden und dass unterschiedliche Druckmaterialien ausgewählt und aufgenommen werden, um das dreidimensionale Druckobjekt (P) mit unterschiedlichen Druckmaterialien aufzubauen und/oder zu bearbeiten.

3. Verfahren nach Anspruch 1 oder 2, **dadurch gekennzeichnet, dass** als Druckmaterial bzw. Druckmaterialien Flüssigkeiten, in Flüssigkeiten gelöste oder suspendierte Feststoffe, Zellsuspensionen oder Biomaterialien eingesetzt werden.

4. Verfahren nach Anspruch 1 oder 2, **dadurch gekennzeichnet, dass** als Druckmaterial bzw. Druckmaterialien einstechbare oder amorphe Feststoffe oder gefrorene Substanzen eingesetzt werden.

5. Verfahren nach Anspruch 1 oder 2, **dadurch gekennzeichnet, dass** als Druckmaterial bzw. Druckmaterialien pulverförmige oder granuläre Feststoffe eingesetzt werden.

6. Verfahren nach einem der Ansprüche 1 bis 5, **dadurch gekennzeichnet, dass** mittels der Dosiervorrichtung (20; 220) jeweils eine quantitativ einem Druckmaterialelement (N0, N1, N2, N3, M4, M5, NM) entsprechende Menge an Druckmaterial (M, N, O) aufgenommen wird.

7. Verfahren nach einem der Ansprüche 1 bis 6, **dadurch gekennzeichnet, dass** die jeweils aufgenommene Menge an Druckmaterial (M, N, O) vor dem Aufbringen auf das Substrat (60) bzw. das darauf angeordnete oder darauf im Aufbau befindliche dreidimensionale Druckobjekt (P) gravimetrisch gewogen wird und dass anhand des Wägeergebnisses und vorgegebener Kriterien entschieden wird, ob die aufgenommene Menge ergänzt oder verworfen und eine neue Menge aufgenommen wird.

8. Verfahren nach einem der Ansprüche 1 bis 7, **dadurch gekennzeichnet, dass** die durch die Dosiervorrichtung (20; 220) in der Zielposition auf das Substrat (60) bzw. das darauf angeordnete oder darauf im Aufbau befindliche dreidimensionale Druckobjekt (P) in Form eines Druckmaterialelements aufgebrachte Druckmaterialmenge durch Applizieren gerichteter Strahlung oder Hitze oder durch ein anderes härtendes oder polymerisierendes Verfahren mit dem Substrat bzw. dem darauf angeordneten oder bereits zum Teil aufgebauten dreidimensionalen Druckobjekt verschmolzen und/oder gehärtet wird.

9. Verfahren nach einem der Ansprüche 1 bis 8, **dadurch gekennzeichnet, dass** die Dosiervorrichtung (20; 220) ein auswechselbares Dosierwerkzeug (24) umfasst, wobei das Dosierwerkzeug (24) vor einem Wechsel des aufzunehmenden Druckmaterials verworfen und durch ein neues Dosierwerkzeug ersetzt wird.

10. Verfahren nach einem der Ansprüche 1 bis 8, **dadurch gekennzeichnet, dass** die Dosiervorrichtung (20; 220) ein Dosierwerkzeug (24) umfasst, wobei das Dosierwerkzeug (24) vor einem Wechsel des aufzunehmenden Druckmaterials gereinigt wird.

11. Verfahren nach einem der Ansprüche 1 bis 10, **dadurch gekennzeichnet, dass** der Aufbau und/oder die Bearbeitung des dreidimensionalen Druckobjekts (P) teilweise mittels eines zusätzlichen, für sich ebenfalls zur Herstellung und/oder Bearbeitung eines dreidimensionalen Druckobjekts ausgebildeten Drucksystems (90) erfolgt, wobei Druckmaterialelemente sowohl mittels der Dosiervorrichtung (20; 220) als auch mittels des zusätzlichen Drucksystems (90) auf das Substrat (60) bzw. das darauf angeordnete oder das darauf in Aufbau befindliche dreidimensionale Druckobjekt (P) aufgebracht werden.

12. Verfahren nach einem der Ansprüche 1 bis 11, **dadurch gekennzeichnet, dass** mindestens ein Druckmaterial so ausgebildet ist, dass das auf das Substrat (60) bzw. das darauf angeordnete oder darauf im Aufbau befindliche dreidimensionale Druckobjekt (P) aufgebrachte Druckmaterialelement (N0, N1, N2, N3, M4, M5, NM) eine materialabbauende Wirkung aufweist, so dass Material aus dem dreidimensionalen Druckobjekt (P) abgebaut wird.

13. Verfahren nach Anspruch 12, **dadurch gekennzeichnet, dass** die materialabbauende Wirkung erst nach Aktivierung des Druckmaterialelements (N0, N1, N2, N3, M4, M5, NM) auftritt, insbesondere nach Aktivierung mittels Hitze oder Strahlung.

14. Verfahren nach Anspruch 12 oder 13, **dadurch gekennzeichnet, dass** das mindestens eine Druckmaterial eine Säure oder ein Lösungsmittel ist.

15. Verfahren nach einem der Ansprüche 1 bis 14, **dadurch gekennzeichnet, dass** mindestens ein Druckmaterial so ausgebildet ist, dass das auf das Substrat (60) bzw. das darauf angeordnete oder darauf im Aufbau befindliche dreidimensionale Druckobjekt (P) aufgebrachte Druckmaterialelement (N0, N1, N2, N3, M4, M5, NM) die physikalischen oder chemischen Eigenschaften des dreidimensionalen Druckobjekts (P) punktuell verändert.

16. Verfahren nach einem der Ansprüche 1 bis 15, **dadurch gekennzeichnet, dass** mittels der Dosiervorrichtung (20; 220) nacheinander unterschiedliche Druckmaterialien (M, N, O) aus mindestens zwei Vorratsbehältern (41, 42, 43; 44, 45) aufgenommen, zu einer in allen drei Raumdimensionen definierten Zielposition transportiert und die unterschiedlichen Druckmaterialien dort nacheinander auf das Substrat (60) bzw. das darauf angeordnete oder darauf im Aufbau befindliche dreidimensionale Druckobjekt (P) aufgebracht werden.

17. Verfahren nach einem der Ansprüche 1 bis 15, **dadurch gekennzeichnet, dass** mittels der Dosiervorrichtung (20; 220) nacheinander unterschiedliche Druckmaterialien (M, N, O) aus mindestens zwei Vorratsbehältern (41, 42, 43; 44, 45) aufgenommen, zu einer in allen drei Raumdimensionen definierten Zielposition transportiert und dort auf das Substrat (60) bzw. das darauf angeordnete oder darauf im Aufbau befindliche dreidimensionale Druckobjekt (P) aufgebracht werden, wobei die unterschiedlichen Druckmaterialien (M, N, O) vor dem Aufbringen in der Dosiervorrichtung (20; 220) miteinander vermischt werden.

18. Verfahren nach einem der Ansprüche 1 bis 17, **dadurch gekennzeichnet, dass** die Dosiervorrichtung (20; 220) mindestens zwei Dosierkanäle (D1, D2, D3, D4) aufweist, mittels welcher nacheinander oder gleichzeitig Druckmaterial oder Druckmaterialien (M, N, O) aus einem oder mehreren Vorratsbehältern (41, 42, 43; 44, 45) aufgenommen und danach zu mindestens einer in allen drei Raumdimensionen definierten Zielposition transportiert und dort auf das Substrat (60) bzw. das darauf angeordnete oder darauf im Aufbau befindliche dreidimensionale Druckobjekt (P) aufgebracht wird bzw. werden.

19. Vorrichtung zur Herstellung und/oder Bearbeitung eines dreidimensionalen Druckobjekts (P) mit einem Druckmaterial (M, N, O), mit einer Einrichtung zur Abgabe von Druckmaterial (M, N, O) in Form von diskreten dreidimensionalen Druckmaterialelementen (N0, N1, N2, N3, M4, M5, NM) an einer Zielposition, umfassend:
- mindestens einen Vorratsbehälter (41, 42, 43; 44, 45), in welchem ein Druckmaterial (M, N, O) vorrätig gehalten ist,
- eine Dosiervorrichtung (20; 220), welche dazu ausgebildet ist, aus dem mindestens einen Vorratsbehälter (41, 42, 43; 44, 45) Druckmaterial aufzunehmen, und
- eine Transporteinrichtung (10), die dazu ausgebildet ist, die Dosiervorrichtung (20; 220) zu dem mindestens einen Vorratsbehälter (41, 42, 43; 44, 45) zu bewegen und zu einer in allen drei Raumdimensionen definierten Zielposition zu transportieren, wobei die Dosiervorrichtung (20; 220) dazu ausgebildet ist, das von ihr aufgenommene Druckmaterial an dieser Zielposition in einer dosierten Menge auf ein Substrat (60) bzw. ein darauf angeordnetes oder ein darauf im Aufbau befindliches dreidimensionales Druckobjekt (P) aufzubringen, wobei die Vorrichtung mindestens eine Strahlungsquelle (80; 81, 82) zur punktgenauen Beaufschlagung der auf das Substrat (60) bzw. das darauf angeordnete oder das darauf im Aufbau befindliche dreidimensionale Druckobjekt (P) aufgebrachten Druckmaterialmenge aufweist.

20. Vorrichtung nach Anspruch 19, **dadurch gekennzeichnet, dass** die Dosiervorrichtung (20; 220) mindestens ein in einem Halter (30) bereitgestelltes Dosierwerkzeug (24) aufweist, welches dazu ausgebildet ist, in einen Dosierkopf (22) der Dosiervorrichtung (20; 220) eingesetzt und wieder aus diesem entfernt zu werden, dass die Transporteinrichtung (10) dazu ausgebildet ist, den Dosierkopf (22) zu dem im Halter (30) bereitgestellten Dosierwerkzeug (24) zu bewegen und dieses in den Dosierkopf (22) einzusetzen, und dass die Transporteinrichtung (10) ferner dazu ausgebildet ist, die Dosiervorrichtung (20; 220) zusammen mit dem eingesetzten Dosierwerkzeug (24) über den Vorratsbehälter (41, 42, 43; 44, 45) zu bewegen und das Dosierwerkzeug (24) in das darin vorrätig gehaltene Druckmaterial einzutauchen, wobei eine definierte Menge des Druckmaterials vom Dosierwerkzeug (24) aufnehmbar ist.

21. Vorrichtung nach Anspruch 19 oder 20, **dadurch gekennzeichnet, dass** die Transporteinrichtung einen von einem Steuerungsrechner (C) gesteuerten Handhabungsroboter (10) umfasst, welcher eine räumliche Positioniergenauigkeit von mindestens 100-200 µm, vorzugsweise bis hinunter zu 1-2 µm, aufweist.

22. Vorrichtung nach einem der Ansprüche 19 bis 21, **dadurch gekennzeichnet, dass** sie mehrere Vorratsbehälter für gleiche oder unterschiedliche Druckmaterialien umfasst und dass die Vorratsbehälter durch Einzelbehälter (41, 42, 43) oder eine Platte (44), die vorzugsweise mehrere Vertiefungen (45) aufweist, gebildet sind.

23. Vorrichtung nach einem der Ansprüche 19 bis 22, **dadurch gekennzeichnet, dass** sie mehrere im Halter (30) bereitgestellte Dosierwerkzeuge (24) umfasst und dass die Dosierwerkzeuge als Röhrchen bzw. Kapillaren (241) unterschiedlicher Grösse ausgebildet sind, in welche Druckmaterial eingebracht und wieder aus ihnen abgegeben werden kann.

24. Vorrichtung nach Anspruch 23, **dadurch gekennzeichnet, dass** die Röhrchen (241) einen in ihnen beweglichen Kolben (242) aufweisen, wobei durch Einsaugen von flüssigem Druckmaterial durch Zurückziehen des Kolbens (242) eine definierte Menge an Druckmaterial aufnehmbar und durch Vorwärtsbewegen des Kolbens (242) aus dem Röhrchen (241) wieder abgebbar ist.

25. Vorrichtung nach Anspruch 23, **dadurch gekennzeichnet, dass** die Röhrchen (241) einen in ihnen beweglichen Kolben (242) aufweisen, wobei durch Einstechen eines Röhrchens (241) in festes oder amorphes Druckmaterial oder Pulver oder Granulat eine definierte Menge an Druckmaterial aufnehmbar und durch Vorwärtsbewegen des Kolbens aus dem Röhrchen wieder abgebbar ist.

26. Vorrichtung nach einem der Ansprüche 19 bis 22, **dadurch gekennzeichnet, dass** sie mehrere im Halter (30) bereitgestellte Dosierwerkzeuge (24) umfasst und dass die Dosierwerkzeuge als Wegwerf-Spritzen ausgebildet sind.

27. Vorrichtung nach einem der Ansprüche 19 bis 22, **dadurch gekennzeichnet, dass** sie mehrere im Halter (30) bereitgestellte Dosierwerkzeuge (24) umfasst und dass die Dosierwerkzeuge als Stäbchen (243) unterschiedlicher Grösse ausgebildet sind, an deren einem Ende beim Eintauchen in Druckmaterial Druckmaterial haften bleibt.

28. Vorrichtung nach einem der Ansprüche 19 bis 27, **dadurch gekennzeichnet, dass** sie eine Heizung (26) oder Strahlungsquelle zum Beheizen des Dosierwerkzeugs (24) im in den Dosierkopf (22) eingesetzten Zustand aufweist.

29. Vorrichtung nach einem der Ansprüche 19 bis 28, **dadurch gekennzeichnet, dass** die mindestens eine Strahlungsquelle (81, 82) am Dosierkopf (22) angeordnet ist.

30. Vorrichtung nach einem der Ansprüche 19 bis 29, **dadurch gekennzeichnet, dass** das Dosierwerkzeug (24) so ausgebildet ist, dass die von der mindestens einen Strahlungsquelle (81, 82) ausgehende Strahlung durch das Dosierwerkzeug (24) hindurch zu der aufgebrachten Druckmaterialmenge leitbar ist.

31. Vorrichtung nach einem der Ansprüche 19 bis 30, **dadurch gekennzeichnet, dass** die mindestens eine Strahlungsquelle (80; 81, 82) für das Schmelzen oder Härten von Druckmaterial ausgebildet ist.

32. Vorrichtung nach einem der Ansprüche 19 bis 31, **dadurch gekennzeichnet, dass** sie zwei oder mehrere Vorratsbehälter (41, 42, 43; 45) für unterschiedliche Druckmaterialien umfasst und dass sie dazu ausgebildet ist, Druckmaterial aus unterschiedlichen Vorratsbehältern auszuwählen und in die Dosiervorrichtung (20; 220) aufzunehmen, wobei das dreidimensionale Druckobjekt (P) aus zwei oder mehreren unterschiedlichen Druckmaterialien aufbaubar ist.

33. Vorrichtung nach einem der Ansprüche 19 bis 32, **dadurch gekennzeichnet, dass** sie mindestens ein zusätzliches eigenständiges Drucksystem (90) umfasst, welches für sich ebenfalls zur Herstellung und/oder Bearbeitung eines dreidimensionalen Druckobjekts ausgebildet ist.

34. Vorrichtung nach Anspruch 33, **dadurch gekennzeichnet, dass** sie dazu ausgebildet ist, das dreidimensionale Druckobjekt (P) teilweise mittels der Dosiervorrichtung (20; 220) und teilweise mittels des zusätzlichen Drucksystems (90) aufzubauen und/oder zu bearbeiten.

35. Vorrichtung nach einem der Ansprüche 19 bis 34, **dadurch gekennzeichnet, dass** die Dosiervorrichtung (20; 220) mindestens zwei Dosierkanäle (D1, D2, D3, D4) aufweist, mittels welcher nacheinander oder gleichzeitig Druckmaterial oder Druckmaterialien (M, N, O) aus einem oder mehreren Vorratsbehältern (41, 42, 43; 44, 45) aufnehmbar und danach zu mindestens einer in allen drei Raumdimensionen definierten Zielposition transportierbar und dort auf das Substrat (60) bzw. das darauf angeordnete oder darauf im Aufbau befindliche dreidimensionale Druckobjekt (P) aufbringbar ist bzw. sind.

## Claims

1. Method for the production and/or treatment of a three-dimensional printed object (P) with a printing material (M, N, O) which is dispensed at a target position in the form of discrete three-dimensional printing material elements (N0, N1, N2, N3, M4, M5, NM), wherein a metering device (20; 220) is moved to at least one reservoir (41, 42, 43; 44, 45) in which a supply of printing material (M, N, O) is kept; wherein printing material is picked up from that at least one reservoir (41, 42, 43; 44, 45) by means of the metering device (20; 220); wherein the metering device (20; 220) is moved to a target position defined in all three spatial dimensions; and wherein, at that target position, a metered quantity of printing material is applied by means of the metering device (20; 220) to a substrate (60) or to a three-dimensional printed object (P) arranged thereon or being constructed thereon, in order to create a printing material element (N0, N1, N2, N3, M4, M5, NM), the creation of a printing material element (N0, N1, N2, N3, M4, M5, NM) being repeated until the three-dimensional printed object (P) has been fully constructed and/or treated.

2. Method according to claim 1, **characterised in that** a supply of two or more different printing materials (M, N, O) is kept; and different printing materials are selected and picked up in order to construct and/or treat the three-dimensional printed object (P) with different printing materials.

3. Method according to claim 1 or 2, **characterised in that** the printing material or printing materials used are liquids, solids dissolved or suspended in liquids, cellular suspensions or biomaterials.

4. Method according to claim 1 or 2, **characterised in that** the printing material or printing materials used are penetrable or amorphous solids or frozen substances.

5. Method according to claim 1 or 2, **characterised in that** the printing material or printing materials used are pulverulent or granular solids.

6. Method according to any one of claims 1 to 5, **characterised in that** by means of the metering device (20; 220) in each case a quantity of printing material (M, N, O) is picked up that corresponds quantitatively to a printing material element (N0, N1, N2, N3, M4, M5, NM).

7. Method according to any one of claims 1 to 6, **characterised in that** the quantity of printing material (M, N, O) picked up in each case is weighed gravimetrically before application to the substrate (60) or to the three-dimensional printed object (P) arranged thereon or being constructed thereon; and, based on the result of weighing and preset criteria, a decision is made as to whether the quantity picked up is supplemented or discarded and a new quantity picked up.

8. Method according to any one of claims 1 to 7, **characterised in that** the quantity of printing material applied, in the form of a printing material element, by the metering device (20; 220) in the target position to the substrate (60) or to the three-dimensional printed object (P) arranged thereon or being constructed thereon is hardened and/or fused to the substrate or to the three-dimensional printed object arranged or already partly constructed thereon by application of directed radiation or heat or by some other hardening or polymerising method.

9. Method according to any one of claims 1 to 8, **characterised in that** the metering device (20; 220) comprises an exchangeable metering tool (24), the metering tool (24) being discarded and replaced by a fresh metering tool prior to a change of the printing material to be picked up.

10. Method according to any one of claims 1 to 8, **characterised in that** the metering device (20; 220) comprises a metering tool (24), the metering tool (24) being cleaned prior to a change of the printing material to be picked up.

11. Method according to any one of claims 1 to 10, **characterised in that** the construction and/or treatment of the three-dimensional printed object (P) is partly effected by means of an additional printing system (90) which is itself likewise configured for the production and/or treatment of a three-dimensional printed object, in which case printing material elements are applied to the substrate (60) or to the three-dimensional printed object (P) arranged thereon or being constructed thereon both by means of the metering device (20; 220) and by means of the additional printing system (90).

12. Method according to any one of claims 1 to 11, **characterised in that** at least one printing material is configured so that the printing material element (N0, N1, N2, N3, M4, M5, NM) applied to the substrate (60) or to the three-dimensional printed object (P) arranged thereon or being constructed thereon has a material-removing action, so that material is removed from the three-dimensional printed object (P).

13. Method according to claim 12, **characterised in that** the material-removing action occurs only after activation of the printing material element (N0, N1, N2, N3, M4, M5, NM), especially after activation by means of heat or radiation.

14. Method according to claim 12 or 13, **characterised in that** the at least one printing material is an acid or a solvent.

15. Method according to any one of claims 1 to 14, **characterised in that** at least one printing material is configured so that the printing material element (N0, N1, N2, N3, M4, M5, NM) applied to the substrate (60) or to the three-dimensional printed object (P) arranged thereon or being constructed thereon modifies the physical or chemical properties of the three-dimensional printed object (P) point by point.

16. Method according to any one of claims 1 to 15, **characterised in that** by means of the metering device (20; 220) different printing materials (M, N, O) are picked up one after the other from at least two reservoirs (41, 42, 43; 44, 45) and transported to a target position defined in all three spatial dimensions and, at that position, the different printing materials are applied one after the other to the substrate (60) or to the three-dimensional printed object (P) arranged thereon or being constructed thereon.

17. Method according to any one of claims 1 to 15, **characterised in that** by means of the metering device (20; 220) different printing materials (M, N, O) are picked up one after the other from at least two reservoirs (41, 42, 43; 44, 45), transported to a target position defined in all three spatial dimensions and, at that position, applied to the substrate (60) or to the three-dimensional printed object (P) arranged thereon or being constructed thereon, the different printing materials (M, N, O) being mixed with one another in the metering device (20; 220) prior to application.

18. Method according to any one of claims 1 to 17, **characterised in that** the metering device (20; 220) has at least two metering channels (D1, D2, D3, D4) by means of which printing material or printing materials (M, N, O) is/are picked up, one after the other or simultaneously, from one or more reservoirs (41, 42, 43; 44, 45) and then transported to at least one target position defined in all three spatial dimensions and, at that position, applied to the substrate (60) or to the three-dimensional printed object (P) arranged thereon or being constructed thereon.

19. Device for the production and/or treatment of a three-dimensional printed object (P) with a printing material (M, N, O), having an apparatus for dispensing printing material (M, N, O) in the form of discrete three-dimensional printing material elements (N0, N1, N2, N3, M4, M5, NM) at a target position, comprising:
- at least one reservoir (41, 42, 43; 44, 45) in which a supply of printing material (M, N, O) is kept,
- a metering device (20; 220) which is configured to pick up printing material from the at least one reservoir (41, 42, 43; 44, 45), and
- a transport device (10) which is configured to move the metering device (20; 220) to the at least one reservoir (41, 42, 43; 44, 45) and to transport the metering device to a target position defined in all three spatial dimensions, wherein the metering device (20; 220) is configured to apply the printing material that it has picked up, at this target position, in a metered amount, to a substrate (60) or to a three-dimensional printed object (P) arranged thereon or being constructed thereon, wherein the device for the production and/or treatment of a three-dimensional printed object has at least one radiation source (80; 81, 82) for high-precision action on the printing material quantity applied to the substrate (60) or to the three-dimensional printed object (P) arranged thereon or being constructed thereon.

20. Device according to claim 19, **characterised in that** the metering device (20; 220) has at least one metering tool (24) provided in a holder (30), which metering tool is configured to be installed in a metering head (22) of the metering device (20; 220) and removed again therefrom; the transport device (10) is configured to move the metering head (22) to the metering tool (24) provided in the holder (30) and to install the metering tool in the metering head (22); and the transport device (10) is further configured to move the metering device (20; 220) together with the installed metering tool (24) over the reservoir (41, 42, 43; 44, 45) and to dip the metering tool (24) into the supply of printing material kept therein, whereby a defined quantity of the printing material can be picked up by the metering tool (24).

21. Device according to claim 19 or 20, **characterised in that** the transport device comprises a handling robot (10) controlled by a control computer (C), which robot has a spatial positioning accuracy of at least 100-200 µm, preferably down to 1-2 µm.

22. Device according to any one of claims 19 to 21, **characterised in that** it comprises a plurality of reservoirs for the same or different printing materials; and the reservoirs are formed by individual containers (41, 42, 43) or by a plate (44) which preferably has a plurality of wells (45).

23. Device according to any one of claims 19 to 22, **characterised in that** it comprises a plurality of metering tools (24) provided in the holder (30); and the metering tools are in the form of tubes or capillaries (241) of different sizes into which printing material can be introduced and dispensed again therefrom.

24. Device according to claim 23, **characterised in that** the tubes (241) have a plunger (242) movable therein, it being possible for a defined quantity of printing material to be picked up by drawing in liquid printing material by retraction of the plunger (242) and to be dispensed from the tube (241) again by forward movement of the plunger (242).

25. Device according to claim 23, **characterised in that** the tubes (241) have a plunger (242) movable therein, it being possible for a defined quantity of printing material to be picked up by insertion of a tube (241) into solid or amorphous printing material or powder or granules and to be dispensed from the tube again by forward movement of the plunger.

26. Device according to any one of claims 19 to 22, **characterised in that** it comprises a plurality of metering tools (24) provided in the holder (30); and the metering tools are in the form of disposable syringes.

27. Device according to any one of claims 19 to 22, **characterised in that** it comprises a plurality of metering tools (24) provided in the holder (30); and the metering tools are in the form of differently sized rods (243), to one end of which printing material adheres when the rod is dipped into printing material.

28. Device according to any one of claims 19 to 27, **characterised in that** it has a heater (26) or radiation source for heating the metering tool (24) in the state in which it is installed in the metering head (22).

29. Device according to any one of claims 19 to 28, **characterised in that** the at least one radiation source (81, 82) is arranged on the metering head (22).

30. Device according to any one of claims 19 to 29, **characterised in that** the metering tool (24) is configured so that the radiation emitted by the at least one radiation source (81, 82) is conductible through the metering tool (24) to the applied printing material quantity.

31. Device according to any one of claims 19 to 30, **characterised in that** the at least one radiation source (80; 81, 82) is configured for the melting or hardening of printing material.

32. Device according to any one of claims 19 to 31, **characterised in that** it comprises two or more reservoirs (41, 42, 43; 45) for different printing materials; and it is configured for selecting printing material from different reservoirs and for picking up that printing material in the metering device (20; 220), it being possible for the three-dimensional printed object (P) to be constructed from two or more different printing materials.

33. Device according to any one of claims 19 to 32, **characterised in that** it comprises at least one additional independent printing system (90) which is itself likewise configured for the production and/or treatment of a three-dimensional printed object.

34. Device according to claim 33, **characterised in that** it is configured for constructing and/or treating the three-dimensional printed object (P) partly by means of the metering device (20; 220) and partly by means of the additional printing system (90).

35. Device according to any one of claims 19 to 34, **characterised in that** the metering device (20; 220) has at least two metering channels (D1, D2, D3, D4) by means of which printing material or printing materials (M, N, O) can be picked up, one after the other or simultaneously, from one or more reservoirs (41, 42, 43; 44, 45) and then transported to at least one target position defined in all three spatial dimensions and, at that position, applied to the substrate (60) or to the three-dimensional printed object (P) arranged thereon or being constructed thereon.

## Revendications

1. Procédé de fabrication et/ou de traitement d'un objet d'impression tridimensionnel (P) avec un matériau d'impression (M, N, O) qui est distribué sous forme d'éléments de matériau d'impression tridimensionnels discrets (N0, N1, N2, N3, M4, M5, NM) dans une position cible,
dans lequel un dispositif de dosage (20 ; 220) est déplacé vers au moins un réservoir (41, 42, 43 ; 44, 45) dans lequel un matériau d'impression (M, N, O) est maintenu en stock, du matériau d'impression est prélevé dans ledit au moins un réservoir (41, 42, 43 ; 44, 45) au moyen du dispositif de dosage (20 ; 220), le dispositif de dosage (20 ; 220) est déplacé vers une position cible définie dans les trois dimensions de l'espace, et dans cette position cible, au moyen du dispositif de dosage (20 ; 220), une quantité dosée de matériau d'impression est appliquée sur un substrat (60) ou sur un objet d'impression tridimensionnel (P) disposé sur celui-ci ou étant en cours de création sur celui-ci, afin de former un élément de matériau d'impression (N0, N1, N2, N3, M4, M5, NM), la formation d'un élément de matériau d'impression (N0, N1, N2, N3, M4, M5, NM) étant répétée jusqu'à ce que l'objet d'impression tridimensionnel (P) soit entièrement créé et/ou traité.

2. Procédé selon la revendication 1,
**caractérisé en ce que** deux ou plusieurs matériaux d'impression différents (M, N, O) sont maintenus en stock, et **en ce que** différents matériaux d'impression sont sélectionnés et prélevés pour créer et/ou traiter l'objet d'impression tridimensionnel (P) avec différents matériaux d'impression.

3. Procédé selon la revendication 1 ou 2,
**caractérisé en ce que**, en tant que matériau(x) d'impression, on utilise des liquides, des solides dissous ou en suspension dans des liquides, des suspensions cellulaires ou des biomatériaux.

4. Procédé selon la revendication 1 ou 2,
**caractérisé en ce que**, en tant que matériau(x) d'impression, on utilise des solides pénétrables ou amorphes ou des substances congelées.

5. Procédé selon la revendication 1 ou 2,
**caractérisé en ce que**, en tant que matériau(x) d'impression, on utilise des solides pulvérulents ou granuleux.

6. Procédé selon l'une des revendications 1 à 5,
**caractérisé en ce qu'**une quantité respective de matériau d'impression (M, N, O) correspondant à la quantité d'un élément de matériau d'impression (N0, N1, N2, N3, M4, M5, NM) est prélevée au moyen du dispositif de dosage (20 ; 220).

7. Procédé selon l'une des revendications 1 à 6,
**caractérisé en ce que** la quantité de matériau d'impression (M, N, O) respective prélevée est pesée par gravimétrie avant d'être appliquée sur le substrat (60) ou sur l'objet d'impression tridimensionnel (P) disposé sur celui-ci ou étant en cours de création sur celui-ci, et
**en ce que**, à l'aide du résultat de la pesée et de critères prédéfinis, on décide si la quantité prélevée sera complétée ou rejetée et qu'une nouvelle quantité sera prélevée.

8. Procédé selon l'une des revendications 1 à 7,
**caractérisé en ce que** la quantité de matériau d'impression appliquée sous forme d'élément de matériau d'impression par le dispositif de dosage (20 ; 220) dans la position cible sur le substrat (60) ou sur l'objet d'impression tridimensionnel (P) disposé sur celui-ci ou étant en cours de création sur celui-ci est fusionnée et/ou durcie avec le substrat ou avec l'objet d'impression tridimensionnel disposé sur celui-ci ou déjà partiellement créé, par application d'un rayonnement ciblé ou de chaleur ou par un autre procédé de durcissement ou de polymérisation.

9. Procédé selon l'une des revendications 1 à 8,
**caractérisé en ce que** le dispositif de dosage (20 ; 220) comprend un outil de dosage interchangeable (24), l'outil de dosage (24) étant rejeté et remplacé par un nouvel outil de dosage avant un changement du matériau d'impression à prélever.

10. Procédé selon l'une des revendications 1 à 8,
**caractérisé en ce que** le dispositif de dosage (20 ; 220) comprend un outil de dosage (24), l'outil de dosage (24) étant nettoyé avant un changement du matériau d'impression à prélever.

11. Procédé selon l'une des revendications 1 à 10,
**caractérisé en ce que** la création et/ou le traitement de l'objet d'impression tridimensionnel (P) s'effectue en partie au moyen d'un système d'impression (90) supplémentaire, réalisé en soi également pour la fabrication et/ou le traitement d'un objet d'impression tridimensionnel, des éléments de matériau d'impression étant appliqués aussi bien par le dispositif de dosage (20 ; 220) que par le système d'impression supplémentaire (90) sur le substrat (60) ou sur l'objet d'impression tridimensionnel (P) disposé sur celui-ci ou étant en cours de création sur celui-ci.

12. Procédé selon l'une des revendications 1 à 11,
**caractérisé en ce qu'**au moins un matériau d'impression est réalisé de telle sorte que l'élément de matériau d'impression (N0, N1, N2, N3, M4, M5, NM) appliqué sur le substrat (60) ou sur l'objet d'impression tridimensionnel (P) disposé sur celui-ci ou étant en cours de création sur celui-ci a un effet d'enlèvement de matériau, de telle sorte que du matériau est enlevé de l'objet d'impression tridimensionnel (P).

13. Procédé selon la revendication 12,
**caractérisé en ce que** l'effet d'enlèvement de matériau ne se produit qu'après l'activation de l'élément de matériau d'impression (N0, N1, N2, N3, M4, M5, NM), en particulier après l'activation par chaleur ou rayonnement.

14. Procédé selon la revendication 12 ou 13,
**caractérisé en ce que** ledit au moins un matériau d'impression est un acide ou un solvant.

15. Procédé selon l'une des revendications 1 à 14,
**caractérisé en ce qu'**au moins un matériau d'impression est réalisé de telle sorte que l'élément de matériau d'impression (N0, N1, N2, N3, M4, M5, NM) appliqué sur le substrat (60) ou sur l'objet d'impression tridimensionnel (P) disposé sur celui-ci ou étant en cours de création sur celui-ci modifie ponctuellement les propriétés physiques ou chimiques de l'objet d'impression tridimensionnel (P).

16. Procédé selon l'une des revendications 1 à 15,
**caractérisé en ce que**, au moyen du dispositif de dosage (20 ; 220), différents matériaux d'impression (M, N, O) sont prélevés successivement dans au moins deux récipients (41, 42, 43 ; 44, 45), sont transportés vers une position cible définie dans les trois dimensions de l'espace, et les différents matériaux d'impression y sont appliqués successivement sur le substrat (60) ou sur l'objet d'impression tridimensionnel (P) disposé sur celui-ci ou étant en cours de création sur celui-ci.

17. Procédé selon l'une des revendications 1 à 15,
**caractérisé en ce que**, au moyen du dispositif de dosage (20 ; 220), différents matériaux d'impression (M, N, O) sont prélevés successivement dans au moins deux réservoirs (41, 42, 43 ; 44, 45), sont transportés vers une position cible définie dans les trois dimensions de l'espace, et y sont appliqués sur le substrat (60) ou sur l'objet d'impression tridimensionnel (P) disposé sur celui-ci ou étant en cours de création sur celui-ci, les différents matériaux d'impression (M, N, O) étant mélangés entre eux dans le dispositif de dosage (20 ; 220) avant d'être appliqués.

18. Procédé selon l'une des revendications 1 à 17,
**caractérisé en ce que** le dispositif de dosage (20 ; 220) présente au moins deux canaux de dosage (D1, D2, D3, D4) au moyen desquels, successivement ou simultanément, du matériau d'impression ou des matériaux d'impression (M, N, O) est/sont prélevé(s) dans un ou plusieurs réservoirs (41, 42, 43 ; 44, 45) et est/sont ensuite transporté(s) vers au moins une position cible définie dans les trois dimensions de l'espace, et y est/sont appliqué(s) sur le substrat (60) ou sur l'objet d'impression tridimensionnel (P) disposé sur celui-ci ou étant en cours de création sur celui-ci.

19. Dispositif de fabrication et/ou de traitement d'un objet d'impression tridimensionnel (P) avec un matériau d'impression (M, N, O), comprenant un organe de distribution de matériau d'impression (M, N, O) sous forme d'éléments de matériau d'impression tridimensionnels discrets (N0, N1, N2, N3, M4, M5, NM) dans une position cible, comprenant
- au moins un réservoir (41, 42, 43 ; 44, 45) dans lequel un matériau d'impression (M, N, O) est maintenu en stock,
- un dispositif de dosage (20 ; 220) qui est réalisé pour prélever du matériau d'impression dans ledit au moins un réservoir (41, 42, 43 ; 44, 45), et
- un organe de transport (10) qui est réalisé pour déplacer le dispositif de dosage (20 ; 220) vers ledit au moins un réservoir (41, 42, 43 ; 44, 45) et pour le transporter vers une position cible définie dans les trois dimensions de l'espace, le dispositif de dosage (20 ; 220) étant réalisé pour appliquer dans cette position cible le matériau d'impression, prélevé par celui-ci, en une quantité dosée sur un substrat (60) ou un objet d'impression tridimensionnel (P) disposé sur celui-ci ou étant en cours de création sur celui-ci,
le dispositif comprenant au moins une source de rayonnement (80 ; 81, 82) pour la sollicitation ponctuelle de la quantité de matériau d'impression appliquée sur le substrat (60) ou sur l'objet d'impression tridimensionnel (P) disposé sur celui-ci ou étant en cours de création sur celui-ci.

20. Dispositif selon la revendication 19,
**caractérisé en ce que** le dispositif de dosage (20 ; 220) comprend au moins outil de dosage (24) mis à disposition dans un porte-outil (30) et réalisé pour être inséré dans une tête de dosage (22) du dispositif de dosage (20 ; 220) et pour être retiré de celle-ci,
**en ce que** l'organe de transport (10) est réalisé pour déplacer la tête de dosage (22) vers l'outil de dosage (24) mis à disposition dans le porte-outil (30) et pour insérer ledit outil dans la tête de dosage (22), et
**en ce que** l'organe de transport (10) est en outre réalisé pour déplacer le dispositif de dosage (20 ; 220), conjointement avec l'outil de dosage (24) inséré, jusqu'au-dessus du réservoir (41, 42, 43 ; 44, 45) et pour faire plonger l'outil de dosage (24) dans le matériau d'impression qui y est maintenu en stock, une quantité définie du matériau d'impression pouvant être prélevée par l'outil de dosage (24).

21. Dispositif selon la revendication 19 ou 20,
**caractérisé en ce que** l'organe de transport comprend un robot de manutention (10) commandé par un ordinateur de commande (C), qui assure une précision de positionnement dans l'espace d'au moins 100 à 200 µm, de préférence de 1 à 2 µm.

22. Dispositif selon l'une des revendications 19 à 21,
**caractérisé en ce qu'**il comprend plusieurs réservoirs pour des matériaux d'impression identiques ou différents, et
**en ce que** les réservoirs sont formés par des récipients individuels (41, 42, 43) ou par une plaque (44) présentant de préférence plusieurs cavités (45).

23. Dispositif selon l'une des revendications 19 à 22,
**caractérisé en ce qu'**il comprend plusieurs outils de dosage (24) mis à disposition dans le porte-outil (30), et
**en ce que** les outils de dosage sont réalisés sous forme de tubes ou de capillaires (241) de différentes tailles, dans lesquels le matériau d'impression peut être introduit et à partir desquels il peut être distribué.

24. Dispositif selon la revendication 23,
**caractérisé en ce que** les tubes (241) comprennent un piston (242) mobile dans ceux-ci, une quantité définie de matériau d'impression pouvant être prélevée par aspiration de matériau d'impression liquide par recul du piston (242) et étant distribuée hors du tube (241) par avancement du piston.

25. Dispositif selon la revendication 23,
**caractérisé en ce que** les tubes (241) comprennent un piston (242) mobile dans ceux-ci, une quantité définie de matériau d'impression pouvant être prélevée par enfoncement d'un tube (241) dans un matériau d'impression solide ou amorphe ou dans une poudre ou des granulés, et pouvant être distribuée hors du tube par avancement du piston.

26. Dispositif selon l'une des revendications 19 à 22,
**caractérisé en ce qu'**il comprend plusieurs outils de dosage (24) mis à disposition dans le porte-outil (30), et
**en ce que** les outils de dosage sont réalisés sous forme de seringues jetables.

27. Dispositif selon l'une des revendications 19 à 22,
**caractérisé en ce qu'**il comprend plusieurs outils de dosage (24) mis à disposition dans le porte-outil (30), et
**en ce que** les outils de dosage (24) sont réalisés sous forme de bâtonnets (243) de différentes tailles, à l'une des extrémités desquels du matériau d'impression reste collé lorsqu'ils sont plongés dans le matériau d'impression.

28. Dispositif selon l'une des revendications 19 à 27,
**caractérisé en ce qu'**il comporte un chauffage (26) ou une source de rayonnement pour chauffer l'outil de dosage (24) à l'état inséré dans la tête de dosage (22).

29. Dispositif selon l'une des revendications 19 à 28,
**caractérisé en ce que** ladite au moins une source de rayonnement (81, 82) est disposée sur la tête de dosage (22).

30. Dispositif selon l'une des revendications 19 à 29,
**caractérisé en ce que** l'outil de dosage (24) est réalisé de telle sorte que le rayonnement émanant de ladite au moins une source de rayonnement (81, 82) peut être amené à travers l'outil de dosage (24) vers la quantité de matériau d'impression appliquée.

31. Dispositif selon l'une des revendications 19 à 30,
**caractérisé en ce que** ladite au moins une source de rayonnement (80 ; 81, 82) est réalisée pour la fusion ou le durcissement de matériau d'impression.

32. Dispositif selon l'une des revendications 19 à 31,
**caractérisé en ce qu'**il comprend deux ou plusieurs réservoirs (41, 42, 43 ; 45) pour différentes matériaux d'impression, et
**en ce qu'**il est réalisé pour sélectionner un matériau d'impression à partir de différents réservoirs et le recevoir dans le dispositif de dosage (20 ; 220), l'objet d'impression tridimensionnel (P) pouvant être créé à partir de deux ou plusieurs matériaux d'impression différents.

33. Dispositif selon l'une des revendications 19 à 32,
**caractérisé en ce qu'**il comprend au moins un système d'impression autonome supplémentaire (90) qui est réalisé en soi également pour la fabrication et/ou le traitement d'un objet d'impression tridimensionnel.

34. Dispositif selon la revendication 33,
**caractérisé en ce qu'**il est réalisé pour créer et/ou traiter l'objet d'impression tridimensionnel (P) en partie au moyen du dispositif de dosage (20 ; 220) et en partie au moyen du système d'impression supplémentaire (90).

35. Dispositif selon l'une des revendications 19 à 34,
**caractérisé en ce que** le dispositif de dosage (20 ; 220) comporte au moins deux canaux de dosage (D1, D2, D3, D4) au moyen desquels, successivement ou simultanément, un matériau d'impression ou des matériaux d'impression (M, N, O) peuvent être prélevés dans un ou plusieurs réservoirs (41, 42, 43 ; 44, 45), puis être transportés vers au moins une position cible définie dans les trois dimensions de l'espace, où ils peuvent être appliqués sur le substrat (60) ou sur l'objet d'impression tridimensionnel (P) disposé sur celui-ci ou étant en cours de création sur celui-ci.
